# EUROPEAN PATENT APPLICATION

(11) **EP 2 484 761 A1**
(43) Date of publication of application: **08.08.2012**
(21) Application number: 10820404.1
(22) Date of filing: 21.09.2010
(51) Int. Cl.: C12N 15/09, C07K 16/00, C12N 5/10

(54) **DNA CONSTRUCT, AND PROCESS FOR PRODUCTION OF RECOMBINANT CHO CELL USING SAME**

(30) Priority: 01.10.2009 JP 2009229722
(71) Applicant: Toto Ltd., Fukuoka 802-8601 (JP)
(72) Inventor: SONEZAKI Shuji, Kitakyushu-shi Fukuoka 802-8601 (JP); OGAMI Yumi, Kitakyushu-shi Fukuoka 802-8601 (JP); YAMANA Yoshimasa, Kitakyushu-shi Fukuoka 802-8601 (JP); NARITA Junya, Kitakyushu-shi Fukuoka 802-8601 (JP)
(74) Representative: ABG Patentes, S.L.
(86) International application number: PCT/JP2010/066317
(87) International publication number: WO 2011/040285

(57) **Abstract**

Disclosed is a DNA construct that is useful for efficient production of recombinant CHO cells useful for the production of target proteins. The DNA construct is a constract comprising, from a 5' end toward a 3' end, a first homologous DNA fragment, a target protein gene, and a second homologous DNA fragment. The first and second homologous DNA fragments have homology allowing for homologous recombination with a part of a hypoxanthine-phosphoribosyltransferase enzyme (hprt) locus in a CHO cell genome and have a chain length of not less than 1 kbp.

SEQUENCE LISTING

<110> TOTO LTD.

<120> DNA construct and a method for preparing a recombinant CHO cell using
the same

<130> P8385EPPC

<160> 91

<170> PatentIn version 3.1

<210> 1
<211> 24
<212> DNA
<213> Artificial

<220>
<223> Designed oligonucleotide primer to amplify a portion of hprt sequence
from CHO-K1

<400> 1
tctgcaggct tcctcctcac accg                                              24


<210> 2
<211> 25
<212> DNA
<213> Artificial

<220>
<223> Designed oligonucleotide primer to amplify a portion of hprt sequence
from CHO-K1

<400> 2
tggaaatggc gttgccttga catgt                                             25


<210> 3
<211> 25
<212> DNA
<213> Artificial

<220>
<223> Designed oligonucleotide primer to amplify a portion of hprt sequence
from CHO-K1

<400> 3
tggaaatggc gttgccttga catgt                                             25


<210> 4
<211> 20
<212> DNA
<213> Artificial

<220>
<223> Designed oligonucleotide primer to amplify a portion of hprt sequence
from CHO-K1

<400> 4
   caccttttcc aaatcctcga                                                    20

<210> 5
<211> 28
<212> DNA
<213> Artificial

<220>
<223> Designed oligonucleotide primer to amplify a portion of hprt sequence
from CHO-K1

<400> 5
agcttatgct ctgatttgaa atcagctg                                          28


<210> 6
<211> 26
<212> DNA
<213> Artificial

<220>
<223> Designed oligonucleotide primer to amplify a portion of hprt sequence
from CHO-K1

<400> 6
cttcagtctg ataaaatcta cagtca
26


<210> 7
<211> 24
<212> DNA
<213> Artificial

<220>
<223> Designed oligonucleotide primer to amplify a portion of hprt sequence
from CHO-K1

<400> 7
aagacttgcc cgagatgtca tgaa
24


<210> 8
<211> 24
<212> DNA
<213> Artificial

<220>
<223> Designed oligonucleotide primer to amplify a portion of hprt sequence
from CHO-K1

<400> 8
ccaagtgagt gattgaaagc acag
24


<210> 9
<211> 24
<212> DNA
<213> Artificial

<220>
<223> Designed oligonucleotide primer to amplify a portion of hprt sequence
from CHO-K1

<400> 9

tgtgtgtatt caagaatatg catg
24

<210> 10
<211> 26
<212> DNA
<213> Artificial

<220>
<223> Designed oligonucleotide primer to amplify a portion of hprt sequence
from CHO-K1

<400> 10
gctgagaaaa tttaacagta ttttag
26

<210> 11
<211> 26
<212> DNA
<213> Artificial

<220>
<223> Designed oligonucleotide primer to amplify a portion of hprt sequence
from CHO-K1

<400> 11
caaatacaag caagaatttc ccagag
26

<210> 12
<211> 20
<212> DNA
<213> Artificial

<220>
<223> Designed oligonucleotide primer to amplify a portion of hprt sequence
from CHO-K1

<400> 12
ggacttgaac atctagggag
20

<210> 13
<211> 24
<212> DNA
<213> Artificial

<220>
<223> Designed oligonucleotide primer to amplify a portion of hprt sequence
from CHO-K1

<400> 13
acttaccact taccattaaa tacc
24

<210> 14
<211> 26
<212> DNA
<213> Artificial

<220>
<223> Designed oligonucleotide primer to amplify a portion of hprt sequence
from CHO-K1

<400> 14
gacaatctat cgaaggctca tagtgc
26


<210> 15
<211> 9638
<212> DNA
<213> Cricetulus griseus

<220>
<221> Intron
<222> (1)..(1)
<223> CHO-K1 hypoxanthine phosphoribosyl transferase intron 1


<400> 15
   gtgagccccg ggactcttca gcagagcggg acagccaggc ctaggcggct ggctgctcag      60
   tggcgccgct cggggcccgt tggcgggcct tgggtgttgg ggcccgcgga agtccggggc     120
   gggggcggtc accgcttccc ggaatccggc tgcgcgtccc cagagggcgg cgggcagcag     180
   gatggaatgg cggggttcag ggctgggtct cccctgggag ccccgagacg gttgactgct     240
   tgtgaggcag gcaggtggaa atggcgttgc cttgacatgt gcggccaccg ggagcccggc     300
   tgggagaggc ggagggcgac ttggagaaag atacctcacg gagaggggag aggggagagg     360
   gcgggggagc tttctcgggg agacccttgc tcttgaagtt cggggcttgg cggccaggga     420
   gagcttcggg gggcgcctct ggaaggagct ccagtaccct acacgacagc atgggagggc     480
   acgttcaggc ctgggctttg cctctaattc tgcagatgtc ataattcgta tttgtggaat     540
   gcgcaattgc tccattgaat ccgcgttgcg gccctgctac tggaaaagca tgtctgtttt     600
   cccacctgag gagactagtg aaaaggcggc tcattttttt tcccacggct tggggttggg     660
   gttgagatac atttgccctt gatgctgtga ggaatttttt ttttaaatat ggatttagtt     720
   gttaggctaa aggacatttc ccccccagtc agcccctgta ttttgtctct tctcccattg     780
   gctctatttt tcgtaacctg acaagctgat cccccactct gctccacaac cttgcttgct     840
   aaaataactt aaagctcctg cctccgatta aatattttag cctgctttat tgtgattatt     900
   gcccctacta ctaattaaat gtcatatcat tctttattaa tatcactgtg gtagatttcg     960
   tagttacctg gttacttgat tgtctctccc acttacacaa actaattatt ttcatttttc    1020
   tggaagggtc ttactgtgta gctctgggtg gttaggaatt ggcaatgtaa accacccggc    1080
   tggtcatgaa cttgtgacag tcctcttgac tcttgcctcc caagtactgc aacgccatgg    1140
   cctgctgagt tttttttttt tcgtggtagg aactgaaccc agagctttgt tcccgatagg    1200
   tgagctgtct acctctgagc caaaggctac cgcccttaaa caagctttat aggtcaggaa    1260
   tcaacattgt agctttagca tctagcacaa tgcctggttt gtggggagct tccattaata    1320
   gtatgttgag tgaattaata agcatttgag cctaaaaatg ctgtctcata gtcatattag 1380
   acagtgtggc ttaaggtaga aactctgttt tttttgtttg tttgtttttt tggattttcg 1440
   agacagggtt tctctgtggc tttggaggct gtcctggaac tagctcttgt agtagaccag 1500
   gctggtctca gaactcacag agatccacct gcctctgcct cccaagtgct gggattaaag 1560
   gcctgtgtca ccaacgcccg ttgaaacact ctgttcttaa tgacctggag tcctgctctt 1620
   gattccccaa gtaaaccagg ccttggattt acagtcatct tgcttctacc tcagtgaggg 1680
   gattacatgc ttgcatgtgc catcttctgg tttgaggagc tggctgctgg tgttgaaagg 1740
   actgtaagac aatatgcaaa taaacaagca tttcacagtt cctgggtgga aacaggaata 1800
   ataaccatca ttgtctttac atagatgatc ctaattagtt ttcagaataa ccctgtaaca 1860
   atggtgtgtt cacattatca gacataatag gggagagacc ccccctcaag tattcagccc 1920
   tggtttagat tagcaccaga agcaaggata aattttaggc ggtggagctc tgccttaaaa 1980
   aatagatcta tctctggagc ctatgggcaa cagtctgctg ttatttttat ttggttactg 2040
   aggagttcta cagatgcctt taagttttga gatttggtac ttgtagagta tagagctaat 2100
   tagttcttaa tcttagaagt gactattctt agttttgttt gaacatcaag aaattcacta 2160
   tttttagctt gcaaattcct gttccttgtt tttaagtttc cttcccctaa attgtctgca 2220
   tgttaggaat aacaaacaca aaaacactta actagtgctt actgtgtgct agttgctttt 2280
   ctaagtcctt taactgactt gatttttttc ctaatttttc tgtatatggc tgttttgcct 2340
   gcatgtatgt atgtaaacca catgcttgcc ctgtgcctgt ggagtccaga agagctcatc 2400
   agatcctcaa gaactggtat ttcagatgat tgtgaactgc caatggaggt tctggaaatg 2460
   aacccaggtc ctctggaaga gtaataagtg ctcttattac tgagccatcc tccaacccct 2520
   acctgactta ctctttaaaa aaatttacac agaggagtta aggaactttg ctaaatcatt 2580
   cagtgccatt acatttgaac ctgttctctg gccccagatt ctacccttaa ccagtaaatt 2640
   taagctagga gctatattaa atgtttctta catacctttc tctgtcttag gcattgacaa 2700
   atgtttttgc agacttcatc taatgtcaat accctaaagt tacataatat ggaccctgtt 2760
   gtgtagacta atatgattgg tgacacaggt agaccaagtg accatgcact agatctcctt 2820
   catgaaactg gcccctaggt gttcctgata gatataaaac acgctgcttg taactatcat 2880
   ttatcaaaaa gcttctggct ttttgcaaac taatgtgaaa catcaggtat gtttcatata 2940
   gcatatcgtt gaatttcaaa atggcccaga tagaagtgtg atttactaac tgaactttta 3000
   cctttctaga tgatttttgt tgttgtcttt tttctgagac atggtttctc tgtggctttg 3060
   gaggctgttc tggaactaac tcttgtagac caggctggtc ttgaactcac agagaaccag 3120
   ctgcctctgc ctcccgagtg ctgggactac aaccacagga cactagatga tttatttttt 3180
   attttttatt tttttaaagt ggcctgaaat tgactatgta gcctaggcct taaactaaca 3240
   cattcctcct gctttaatat tctaagtttt aggattacaa tcatgatcca ccacacttgg 3300
   cttcagggtt tggttttatt tatttgtttt tgtttatttg gtttttcgga gacagcattt 3360
   atctgtgtaa cagccctggc tgtcctggaa ctagctctgt agatcaggct ggccttgaac 3420
   tcacagagat atgcctggag tgctgggatt aaaggcatgc accaccacct ggccctaaag 3480
   gtattttgag atagggactt tagttatagg ctggtctccc aattctcagt catcctacct 3540
   taccctcctg agtgtgagat tagaggtaaa catattccac catgcctagt atgctactca 3600
   gagagtgagg gaaggggagg gagagaggga gtgagttttt gtgatctaac tcaggatctt 3660
   acatattaag catatgcttt attaccattg ggctatacct ctatccatct gatacttttt 3720
   tcgtttgtta agatttattt ctattttgta tatatgagtg ttttccctgt atgtatgtat 3780
   gtatttttta aagattttat ttattatgta tacattctac ttcgatgtat atctgcacac 3840
   cagaagaggg caccagatct tataatggat ggttgtgagc caccatgtgg ttgctggtaa 3900
   ttgaactcag gacctctgga agagcagctg gtgctcttaa cctctgagcc atctctccag 3960
   ccctgtatgt atgtatgtgc atgattagca tgcctgtgga ggtcagaaga gagcacttga 4020
   tctcctagaa ctggagttac agacaattgt gagcttccag gtgagtgctg ggaaccgaat 4080
   ccatgtcctc tgcaaagagc aacaattaca cttaaacact gagccatttc cagcctgttc 4140
   cctccctcag aattggttaa aatattatat atttttgctt gtcactttta ttattatcaa 4200
   cagtgtttaa aataacactc atttaagtaa gatgaaaatt aactatttat gatcaccaaa 4260
   aaatggaaac agttcctctt agcgatttct ttctgtaaat tttgtactgt gttacctata 4320
   catccatctt aattatttta tgttccagct actgtagcag gtaggaaaag aactaatcat 4380
   atcagaaaaa tgtgccttat agttttatta ttgttgtttg gggtggatgg atttctttat 4440
   tagagggttg gtttatttta gtttgaggca ggttttcttt atataggcca ggctgagtct 4500
   ctttaaatgt tgggactata ggaatgcgct gccatgccca gctcttgagg tttctagttt 4560
   attttgtttt caagtcaggg tttctttatg tagccctggc tgtcctggaa ctcactctgt 4620
   agaccaggct ggccttgaac tcatagagat cctcctgcct tgctgtgctg tgctggggtt 4680
   aaaggttggt gggtgtgtgt gtgtgtgtgt gtgtgtgtgt gtgtgtgtgt gtgtgtgtgt 4740
   gtgtgtgtgt gttttgtgtg tctgtgtgtg tgtgtgtgtg tgtgtgtgtg tgtgtgtgtg 4800
   tgtgtgtgtg tgtgtgtgtg tgtgtgtgtg tgtgtgtgtg tgtgtgtgtg tgtgtgagag 4860
   agagagaggc tggccttgaa ctcatagaga tcctcgtgcg tgcgtgcgtg cgcgtgtgct 4920
   gcctctctgt gtgtgtgtgt gtgtgtgtgt gccactactg cccagccatt ttattttatt 4980
   aattttattt atcatgtata cagtcttctg cctgcatgcc agcagagggc accagatctt 5040
   attcatggta gttgtgagtc accatgtggt tgccgggaat tgaactcagg acctctggaa 5100
   gagcagtcgg tgcacttaac cactgagcca tttctccagc ccagccatct tattttttta 5160
   ctttaaactc ttttagatta agggggaaaa acagctaata tgttttatta ttacaataac 5220
   tgactgtcat gaactgagtt ttcattttct ttatttttat ttttgtgcag agttggtaac 5280
   agataccttt acaaggtttt ttaaatttca cccatttttt ttttaaaatt cacttttatt 5340
   ttttttgttt tgttttatga gacaggctct ctacatagcc ctggatgtcc tggaactatg 5400
   cagatcagca gccggcgttg aattcacaga gatttgcctg cctttgcttc tagtgctggg 5460
   attaaagatg tatcatttgt cactacattt ggcaaatgtt taaaaaaatt aaattttaaa 5520
   tagaattata tccctttttc ccttttcttc ctccagccct tcccatttac gctccgtaaa 5580
   atccctccag tgttcaccct ctcaagttga tagcctcttt ttctttatca ttgttaaacg 5640
   catatattct tgtttgtgtg taaatggttt caaggtttat tacgtggcag tgaacatccg 5700
   gtaaggggct ctttgtggaa gcagccattc ttcacccagc agttattagt tacctgtact 5760
   ttgtctaggg gtgggaccca gagaaatttc caaaacttgg aatcctcctg ccttagtttt 5820
   ctgagtgctg ggattatagg catgcactgt cctgcctagc tctagtagtt tttacttatt 5880
   tttagtttga tatttgagac agcatctcat tatacagccc tggctgtcca ggaactctgt 5940
   gtaaaccagg ctagcctcag gcctcaacag aaatttatct tcctctgcct tctaagtgct 6000
   gggattaaag gtatgcacca atgcaccacc acaaacacca agctttaaat aattttttta 6060
   agagatggga tctggctgtg ttgcccaggt tacccctgcc ttaatcttct acgtagttag 6120
   gatcccagtg gaatgttttt tattcttcca taattagtca atgatccatg tcattttttt 6180
   ctcccctata ctaaaagggt actagaaatg tgttcagtct ttctgtaatt ttaagttatg 6240
   tggattcact aacattgata gatcacataa aagtgttaat tactaccaat aacagtaata 6300
   ttgagactca ggtggtattt tcctgttggt gactgccaaa ttactaacat ttaaataatc 6360
   tgctaacatg aattcagaca cttcaaattg ctatgaaacc atcttcaggt ttttgttgta 6420
   tgtttcagta tgaacatacc tattattcta ctatgcacat tagtaaaagg aaaaattatg 6480
   gtattcatgt ccttacagat atggtagtta taggacccat ttgcctagag tggttataat 6540
   atattcctgg gatgaacact ccactacctt atttattttt cttttagttt tagcccttgg 6600
   aagggaaaca aacagtagat gtattatttg taaactgata aaaaattgta agataaacac 6660
   agtatttaac ttgttttcac atttaccagt gtctgtcaat aaattattat aattggttta 6720
   atcataacca gagagtataa attatctcaa gaggatagca tatcatttac tttaatattt 6780
   ttggaaaatt ttaatatatt tgaattaagc tagtgtctca ctatgtagcc aaggctggcg 6840
   tcaaacttga gtcttctgag tgctgagatg ataggtatgt gccaccatgc tcagcaaaat 6900
   cgctattatg tacacattta taataatgga agtcacataa aaattcttgt ggtttacaca 6960
   taaaatttct tgttacacag atatattatc aaaatgatat aaaaagggaa atgaacctaa 7020
   gctaatatga tctttggaat atcaattcat catccacaac taggattgtt taggtctctt 7080
   tgtatttaaa aaatagcatt gttcatgagt ccaaacaagt ggaaatgagt tctgaaatga 7140
   actatgtgtg ttgaacatgg tagtaaagcc ctgtaatcac agcacttgga ggcaaaggca 7200
   ggaggatggg gtcagtcatc ctcagctaca taatgagttc aaagccagcc tgagctcatg 7260
   caactgtttc aaaagagaaa atgattgtga gttaagacat gtataatact aatacaggtg 7320
   tagtgaaaca tacttgtaat cccagtgctt ggaaggcaaa ggcatgaaga ttgccaagag 7380
   ttctaaccca gcctaaacta cattagtggt gagacaggaa caagaggagg tgagaataat 7440
   agataaacag aaaaatgtag ttttataact aataatcaac atgtcccaac ttctataatg 7500
   gaaagaaatt gttaggtgtg gcctccacat atgtgcacct gctttcacac atgcatacac 7560
   ccacatgaat acaaacacaa atatctcagg tgataggaga cataagactt gatttttgcc 7620
   ttttggggaa gaattgtatc aatatggtct taataaactg actatgtggc ccatacctgt 7680
   gaaccccttc atttgggaag ttgagggaga gtgccctagg tgtgcagata ctttgggctg 7740
   tgttgtgaga ttcagattac cctggactac agagctgaga ctgaagtcat caaaaacaag 7800
   atttttactc ttaaaataac tataaatatt atcaaaagca caaaataaaa atcctctgac 7860
   ataaatctgt tttaattaga aattaaaagt tttagattag aaaccagttt tgtgtgttga 7920
   cagaaatagt attcctattt tgaagtactt cctggtgttc tgtttggatt ctttgaatac 7980
   atgactggga gtagggtgag ttggtgtata cctgttatct cagcactcaa gaagcagagg 8040
   caggaggatc atcgcaagct tgaggcagcc tgatctatat atctagttcc aggacagctg 8100
   gagttacata gaagaccctg tctcaaacac ataaaaagac tgggatgaac ttggcagaat 8160
   tcacattgaa tttgtctttg aaacaaacaa tcctttaagt tcttaagtta ccagtacaac 8220
   ctgtaaccag gcacagggcc atttaagtag gtgcagtagt ttgacaaatt ctgggtatga 8280
   tgtggtttaa acttatggca gaggcagtgg gtgggtgaag gggatgatta acctcaagga 8340
   agtcattgtg gtattctaat cttgattcta aagtactttg aaacaatcca acttagtttt 8400
   tgtcagattt tagtgcacac gttctgatga gtctggtagg tatgtttata ttataatatt 8460
   gagaagtctg tcatcctgtc tcaaaacatg aactaaattt tgctttataa aacttataaa 8520
   aatgcgttca taggtgggca atggtggcac acacctttaa tcccagcact tgggaggcag 8580
   aggcaggtgg atctctgagt ttgagaccac tgtggtctac agagcaagtt ccagacaggc 8640
   tccaaatcta cacagagaaa ccctgtctca aacaaacaaa caaacaaaaa agctttcttg 8700
   taaaatactc gttgaatgat cctttttatg atacaaatgt ccaagttcca tttttgttag 8760
   taaaggtatt cttttttttt cttgagacag ggtttctctg tggctttgga ggctgtcctg 8820
   gaactagctc atgtagacca ggctggtctc gaagccacag agatccacct gcctctgcct 8880
   cccaagtgct ggaactaaag gcatgcgcca ccaatgccaa ggtgagtttt taaaggatca 8940
   attaggtttt tggtgacccc ccaccaccac acacacatat atactctgag ataagcattc 9000
   ttaggatttg gatggttaac accagttttc attttctgtg tgagatttta tttaatttac 9060
   aagaatatac tgcattatat taaatatttt aggggattgg gaagatgaca cagtctataa 9120
   agtgcatttc atgtcatctt cagaacctga gtttggaacc cccagcacct agctaaaaag 9180
   caggacattc ctataatccc agcaccaggg aggcagagac agggggatgg atccctgggg 9240
   cttcctgcca aactatacta gccaatgaga gacctggtct caaaacataa atgaattttt 9300
   tagacccatt ttcaatctct ggagttgaca cacatatgca cttgcatttg cccccacaat 9360
   gctcaaacat actcaatgtg tagagtagaa actgcgtaga ctagtctctg atgtttaaag 9420
   tatttttgcc acatttgtca tacctcttta aattgttatc tttttctact tgtgacctga 9480
   aggaagagga ttatagttta ttacaaaaat ataaaattaa ctgcccttca taccctctca 9540
   tacccaaatt cctctggcga actaaggctt attttctaaa ttttgttaaa tatggatatc 9600
   ttatgctctg atttgaaaca gctgtctttc tttttcag 9638

<210> 16
<211> 2539
<212> DNA
<213> Cricetulus griseus

<220>
<221> Intron
<222> (1)..(2539)
<223> CHO-K1 hypoxanthine phosphoribosyl transferase intron 2

<400> 16
gtaagtaaga tcttaattga aggggctgga gagatggctc agcggttaag agcactgact 60

gctcttccag aggttctgag ttcaattccc agcaaccaca tgatggctca caaccatccc 120

taatgagatc tggtgccctc ttctggcctg cagggacaca tgcagacaga acagatctta 180

attgaagttt tatgggttta ttttagatgt gtctgtgtgt atgtttgtga taggcatgta 240

tttgatacat atgtataatg tatatattta tgtaaaagct agagaaggac attgggtgcc 300

atactctgtt gctctctgtc ttgctccctt gagttggtgt ctctcactga acctggggac 360

atgctgattc ggttaggctc tgtcccctat tgctgaagtt tcaagcagat gcggccacac 420

ctggttttta tatgggtatt aaggatctga atttaggtcc tgatgcttgc acaagtgctc 480

ccaatggcct gcatgtatgt gcagcatctg cattccagga gaccacaaaa gtgtgatggg 540

tcctctggaa ctggagttgc aggccattgg gagctactgt gtggctggga actggactta 600

gggcctctgc ccaaacagca aatgggctta actctctcca gccccaatat tttctagttt 660

taaagttctt tttcacaaaa agaaaaacac caggaagtcc tactagttat tcagtttgag 720

atataaatag tggtactcca taatgagtcc agcaaatgtt gaaacgaggt tggaagtctg 780

taaatgagat tatgattctg ttggtttgct atacacattt cttttaaaat ttttttataa 840

attttattta aattagaaac aatcttattt tacatatcaa tcccagttca ctctcactcc 900

catcctccca agccccccac caattctccc atccactccc caaggagggc gaggccttcc 960

atgagggatc atcaaaaaat ctgtcacttg gggcagggcc taggccctcc tcccctgtta 1020

tacacatttc tttactgtat cactgtatgt aaagtatata tacacatacg agttatgtac 1080

tatcatataa tactaaattt ttggactttt taatattagt acatctatag ctatcacatg 1140

tttttatctt taaaaatttt tataattgtg tgtgtgtgtg tgtgtgtgtg tgtgtgtgtg 1200

tgtgtgtgtg tgtgtgtatg tatgtgtatg tgtgtgtatg tgtgtaaact gttttctctt 1260

tgagataggt tcttactgtt tagttcagtc tggcttggaa ctttctgtat agcccagact 1320

gaccttaact cagtattcct cccccatctg gagattacag gtatgagcca ccatgcccag 1380

tcctgttttt atattgattt tgaaaaaggg tctcaaatag tttagactgg cttccaactc 1440

acagtgtagc tgaggatgac ctgaagttct gatgctattg cttctactgg tgtgccatta 1500

tgcccagttt gcacagttat gaggataaaa cctaggacct tgttcctgtg catactaggc 1560

atagactcta ccaattaagc tacattccca gctcccccag gtttacttta aaggtgataa 1620

tgctattctg ttcatggata taccataact gatttaactc tgtactgttg gtagacactt 1680

aagatctcca ttattttgca cttagcagga gtttattaga ggaaatatga tatatattgg 1740

cacttgctca gctttaggca agtagaaatt ctgggtcaag taaaagatgt ttattgaata 1800

ttatcatact gccaaattaa cctctaaaaa atttcataat tttatactac cagtggtatg 1860

tgagttcatt tttcctcaca atctcttcta atattttaca ttagagaaat gcaatttaac 1920

caaaattcac cagtcttgtt agctttgaac attgtatctt tttatttgta tttttaaatt 1980

tcatgatgtt atagtatacc tgttctttta tttgagacag aatcttatta tgtagcccta 2040

agtgacctag tacttactct gctgttgtac agtatgtccc tcaacccata cccttcctcc 2100

tgcctcagcc tccagagtgc tagaattata ggcatgtacc atggtgccta gcatgtacct 2160

ggtttttaaa ggtagctgga attataggca tgtaccatga tgcctagcat gtacctggtt 2220

tttaaagtta gtaagtttta attttggttg agctgtgtgt tggtgtgtaa gtataatctc 2280

agcacacaga gttcgagaca tgggttatga gttactatgc agcctgggct acatagaggg 2340

atcctgtgtc accttcccca acccccaaaa tcttcccttt gccatatgga aaacatcccc 2400

cactttattt aatagtttga tttatgaagc aagattacca attatgggga caaagaatgt 2460

gtcctgtgga agtttaagaa gtgtttgtta taaaaatata actatttgga atcttctatt 2520

cctgatttta tttttgtag 2539

<210> 17
<211> 6665
<212> DNA
<213> Cricetulus griseus

<220>
<221> Intron
<222> (1)..(6665)
<223> CHO-K1 hypoxanthine phosphoribosyl transferase intron 3

<400> 17

gtaagtataa ttcattcata atttaaaaaa tatggcaatc ctagttttgt atgtattttt 60

gtttgtttgt ttttactttg aaacagtgtt tttctgggta gctttggagc ctgtcttgga 120

actctctgct gtagaccatg actgacctac agagatctac ctgcctctga ctcctgagtg 180

ctgagattaa acgagtgcac caccacctga gcctcatcaa ctgaggctta tcccagtttt 240

ttttagaatt ttagtttgag accctctcta acttgctcag actatccttg aactcattac 300

ataggctgat ctagaaattt tagtcatctt gactcagttt ctcaagtagc tgggattaca 360

ggcaggtgct accacttctg gccataattg ttgctgttat tattcactgg tactttacat 420

cacaacactg tagttcaaga ccaacatcaa agtgaatatt gtttgttagt cacccaagat 480

ggatttgtac ttactttgca tggtttttct tttgtcttat tttgacctgg attttgactt 540

gttttatgat actgctcagg ctagtatcaa actctgtggt tcaaataatc cttctgtctt 600

agcctcccaa attctgggag tacaagcgtg tatcaccata cctgactgtg ttttgatatt 660

ttccaaaaca acttttaaga attactgcag agtacagtga cagagagaga tggttcatca 720

gtcacaagca cttgtagaga agactggggt ttagttactg gcacccaaaa gatggctcac 780

agctatgtag ctccagttcc aggggacctg atgtcctagc ctccagataa aataaaaata 840

agtaagtttt acaagaatta tgggactggt gaggtggact cagtagtcaa aggcactggc 900

tgctctttca gaggacctgg ttcaattcct agcacccacg tggtagttca cagttgtctt 960

taactccagt cccagggatc tgatgccatc ttctggcctc caagggcacc agacactaac 1020

atggtacaca aacaaatatg caacttgaac acccatacac atattttaaa aataaacaat 1080

ttctgttgag acttggtgtt ttcctgactg tatatgctgg tggcaaataa tttctctgga 1140

actaagttgc tttgagagta atcctaaatt atttgaagca aagtctcatt attaaggtta 1200

acaattattt ctcagaaaac agaaaccact tttgaccttt ggccacgtta cctttgtcag 1260

agtctgaagt gatgtagaat attgacaata tgatgaaaca aaatgaatgt aatctttaca 1320

tgtaaattcc atcattcaca attttgattg tgtggcttat tttttttatt ttcattctct 1380

ctcttttttt ttttgagact ctatgtagct cttactgtct tggaactcac tgtatagacc 1440

aggctggtct tgaactcaga gacccaactg cctctacctc ctgagtgctg ggattagagg 1500

catgtgccac cactgcctgg ctaaaataga tatttatttt taaaataaaa ctgcaggata 1560

ttatagagaa acttaagtca cccatattct tttgtttttt tgtttttttt tttgtttttg 1620

gtttttccag acagggtttc tctggctttg gaggctgtcc tggaactagc tcttgtagac 1680

caggctggtc tcgaactcac agcctgcctc tgcctcccga gtgctgggat taaaggcgtg 1740

ggccaccaac gcctggctct ctttttggtt ttttaagaca ggatttctct atgtagcttt 1800

ggagcctatc ctggcactcg atctggagac cagactggcc tcaaactcac agatatctgc 1860

ctgctgagta ctgggattaa aggcgtgtgc caccaacgcc tggctaagtc acccatattc 1920

tattttttta agttttattt ttattttata tttatgagtg ttttgcctac atgcatgtgt 1980

gtttaccata tgagtgcctc atgtctacag gtgccagcag aagacatcag atcccctggg 2040
actggagttt cagatgtttg tgagtcaaca tgtaggtgct aggaactgaa cttgggtact 2100
ctgcaagaat agcacataca tttaactatt gaaccatctc tccagctctt gtcaccactt 2160
tttgagtatt ttaaagtgtt ttctgtctgt atacatgtac acaatgctac tatagtttat 2220
ataatcagtg ttatcccaga tgtcatttaa ctaggtggaa aagaacctgg gggttaccaa 2280
atatgtattt cattaaatga aagagtctgc taattaaaac ctctgtattt tttgtgcttc 2340
taagaaagaa aatacctcca gttatagtag catggtacca tgaattatgt tttcagaggc 2400
agtaaagtat gaatatatat cttaaaacag tgaaatatag tgttatattt ggtgacattt 2460
tttggtcccc tatgctctat aaccttcagg aaaatgaaac atttttacaa gtatacaaat 2520
gtgtttcttc tattttgagt tttatttcaa ttttataaaa ttgcactaat agttgatgaa 2580
aataatgagg ttttgggaga aggggtgtta gcaaaccatt attttaagtt acatttttag 2640
atttgtaatt aatttatttt cagtgttgaa tgaatccaag acttcaagta tgctaagcaa 2700
ttacgttgct acttagggat tttcaaagat cttagggatg gaaaattagg aataaacagg 2760
tagacaacgt agaaataaat gttaaaggct gtattttaga cccttgagat tctctgggaa 2820
ttaagagatt tttatgtgta tgagtgtttt gcctgcctga agtctacaga ggtcagaaga 2880
gtgtctgact ttctagaatt ggaattgcat acaattgaga cctgccatgg gtgtgcttgg 2940
aattgaacct gggtggtttg gaagccaatg ctctgaacca cctctgggaa tttttggtta 3000
ctgacagtga cacctaaatg aaggaaccct tgggcactct gcttaatgaa atgatgaaaa 3060
gggaaagaag agatggagcc aatctggaga gaactgtcct ttcattcgtt ccagtcatgt 3120
ctccatagag tttattctaa tagttaaagg tgaggcctct ctgagagagg cactaactta 3180
aggatacagt cagttccaga tacagggagg ttgctgtggt ttttctgccc actcaagcta 3240
ggtaattgat tcatggatag catttgtaaa caatataggc atttatatat aacaactatt 3300
aaatgaacat tttattagcc ccattcaaaa ggaggctgtt ttccttggaa aaagtttcaa 3360
taaaacataa gctgaaacag tgaggtcagt gggactgtgg aaatttgaac agcctttttt 3420
tggtttttcg agacagggtt tctctgtagc tttggaggct gtcctggaac tagctcttgt 3480
agaacaggct ggtcttgaac tcacagagat ccacctgcct ctgcctcccg agtgctggca 3540
ttaaaggcgt gcgccaccac agcccggcct tgaacagtct taagagtgcc aatgttctgt 3600
gtgctttgga gttctgttgc ctcggaccct atagccatag cctttggatc acagcttcat 3660
tcttaggtat ttactccttt tgtcctttcc ttgaaaaggg aattaattgg tgttggactg 3720
taataccatg tttctacaca agatgtcagt atttacctag gtttgttatt actatgtagc 3780
actttagctg taaactacag ctgaaaacct ataaactagt ttcaggtttt ctacaattat 3840
ttttcttttt gtcattaatt agttctgtgt gtttgagatt ctgggtttta gattagagta 3900

ataatatgca aaattacatt aaaaagccaa acatttagtt tacagtatgt actagtagta 3960

tgcttttatt tattttgttt tgtttctttc ttcccacttc cccttacaca taccagacaa 4020

acattctacc aattgaatta tcatttgttt tgttttgttg aagtaagagt ttctgtgtgt 4080

aatcttggct gtcctggaac tggtgctata gaccaggttg gcctagaact cacagagatc 4140

cacctgcctc tgattcccca gggctgggat taaaggcatg agccaccacc acccagctat 4200

cttttttaat gatattttga gataggatct tgttaggtag cccagagtga tctcaaactc 4260

tcaaccctgc cttagcctcc atcctggaac acgctctgta tgccatgcta ggcttaaact 4320

cacagaggtc tgctgtcctc tgcctcccaa gagtgttgag actaaagatg tgcactacca 4380

ccaccaccac ctggcccaac atacttttgt tgtgttttgt ttttcaagac agggtttctc 4440

tgagtgacag cccttgccat cctggatcca cctttttctg cctccctagt gctgggatta 4500

atggcttgta ccaccaccat ctagcctaac atacttttaa atacagaaca aaaatagttt 4560

tataggtagc atatttgtct gctacttggt attattagtt aatattgatc catataagta 4620

tacattaatt ttttgtgatt ctttttaata gttccatagt attattgttg tgtacattaa 4680

cttgatttat ttagttaatc ccctgttacc acatgttgcc tctttttgat gaaaaaagaa 4740

tacagtgggc tgggcgttgg tggtgcacgc ctttaatccc agcactaggg aggcagaggc 4800

cagcctggtc tccagaatga gtgccaggat aggctccaaa gctacacata gaaaccgtgt 4860

ctcgaaaaac caaaaaaaaa aaaaaaatac agtgggtact ttatgttttt tgcatgtttc 4920

tgaatgtttt tgtgacattg attcttggaa atggggttgt tagaccaaaa tggtgtgcat 4980

gtttccagtt ttagtacaca ttgctgtatt cttttcaaaa gaggtcatat tggtttctgt 5040

actccaacca acaacttgag tatttgtttc ccttcaatgt attactagga tttagttctg 5100

aaatgtggat gggtgaacac tggaatcaca tttttttagt agtgcctttt tttgttttgt 5160

ttttcaagac agcatctctc tgtgtaaaag agctctggct gtcctagaac ttgctctctg 5220

taaaccagac tggcctgaac tcgtatagat ccacctgcct ctgcctttcg agtgctggga 5280

ttaaaagctg agctgccacc attaggcttt agtagttcta ttttaaaaag atatatttat 5340

cttactttat gtacatgtgt gaatgccagt atgcatacca tgtgcatgct cgatgctttt 5400

gaggctagaa gggggattag aacacttgaa actggagtta caagcaatta taagctgcca 5460

tgtggatgct gggaactgaa cccaggtcca ttgcaagatc agcatgtgtt cttaactgat 5520

gaactatctc tccagtccca tctagtgggt tcttattttg aagtataaca tgcattaaga 5580

atattggtta tagatatttt cagttggggt tcatgacctg ttttttgccc tttctagtaa 5640

tgcaaaaaaa aaaaaaacaa aaacaaaaac aaaaagtatc ttactacatt tacttccaat 5700

tcacttatag ttttattttt tgtatttaga ttttaatcca tctggaattt atttttgtgc 5760

atggtgtgag gtagggacca tgctttatat tttttctcaa atggataagt aattgaccag 5820

atgccatttg ttgaataatt catcttttaa ttactgactc aagatgccat ctttgtcata 5880

tactaaattg ccatatgttt ttgggtctgt ttctggattt tactttgttc ctttaacttt 5940

ttgtcaatca ctgtactgat tgacacaagg gaattactgt agctttatgg tacttgtcat 6000

tttcctagta gagaaagtcc aggcttttta tttatctgct ttcaattcag ttttctgtct 6060

tcatttcagc acatccaaat aacagttatt cccaccaact acagtgagtg ttaagtgctc 6120

tcttttgaaa cccctataag ggtttctgct aatttctatg agagcagagt gaaaacctaa 6180

gaagtgtgtt ctaacagact ggtactcaga cctggtttct cagttctgaa gatgacttgg 6240

gtatgggctg gactttgagg gggcatatcc tcagggttgg atgtgataca taccagctcc 6300

ttcaacttgt gactcacagg aatgttacag ggccagaagc agacacctgc catgctaata 6360

aattgtcctt tgtggttgag aactgcctga ttgatgatga gcagctatat tatgctcact 6420

cagaaatgga tgaactctat ttttaggact cttcagaaca ttaaaaacca gcaatggcca 6480

gttatgattt ttaaatttta ctaatctcta tttacctagt cattaatttc tagaaaccta 6540

atacttataa tacatgattt agaataccat aatgttgtag ccattagata gctaacttct 6600

tacatctaat aattttttgt gtgtattcaa gaatatgcat gtaaatgatg ttttttttta 6660

actag 6665

<210> 18
<211> 4473
<212> DNA
<213> Cricetulus griseus

<220>
<221> Intron
<222> (1)..(4473)
<223> CHO-K1 hypoxanthine phosphoribosyl transferase intron 4

<400>\201@18
gtaagtacct taaagggaaa gcaaagaatc atttttatac caagaatcaa ttagtaactg 60

tgctttcaat cactcacttg gagatacttt aagtaagcaa ttattgttta atacttgcaa 120

agtaccctta tatagtttta tatatcttct ggactatata gccaaatgac agcagaatct 180

aatagtaaca ctaaaaatcc acattgttaa ttactgcgtt ctgtaatacc tctttgaaaa 240

tctaatttgg tatccttcag aaagtacaaa tggggagaaa ggcagtcttt taaaaagttt 300

tatgccggga ggtggtggca catgccttta atcccagcac tctgcccagg cagaagcagc 360

ctggtctaca gagtaagttc taggacagcc agggctacac agagaaaccc tgtctcaaaa 420

aaaaagttgg ggggggagtt tcacatgtag ttttattttt ttaaagattt tatttattta 480

ttatgtatac aacattctgc ttccatgtat atctgcacac cagaagaggg caccagatct 540

cttaacggat ggttgtgagc caccatgtgg ttgctgggaa ttgaatcagg acccttggaa 600

gagcagtcgg tgctcttaac ctctgagcca tctctccagc cccgtagttt catttttaag 660

gtgtgtgtgt gtgtgtgtgt gtgtgtgtgt gtgtgtgtgt gtgtgtgtgt gtgtgtgtgt 720

gtgtgtgtgt gtgtgtgtgt gtgtgtgtgt gtgtgtgtgt gtgtgtgtgt gtgtgtgtgt 780
gtgtgtgtgt gtgtgtgtgt gtgtgtgtct gtgtgtctgt gtgtctgtgt gtatgtatgt 840
gtatgacgtg catgagtttg ttgagttcag gagccagttc tttccagcat gtagaattga 900
gagttgggag tatgttaaca tgatcaaaat tggcaccaag tacctttact ccctcagcta 960
tctcactttg tagcccatat catcccaaca tctcctcctg cctcagcctc ccaagtgctg 1020
ggattgcaag catgagccac tatactggac tgaatatgtt atactaagct gctagttatt 1080
tacttcagat actgtgcctt gtataaatgt attactttgg taaatttcac agtaatctgg 1140
taagggaagt tatattctca ttctgcagat caggaaatta agcaatatag ttacagtttg 1200
gaaaaataca tgggattatg agacaactgt ctcacaatct caaagtcaca cagccaggaa 1260
gtagaagagc ttgaatcctg acatcagagt tctttcttgg tctagttttt acattttagt 1320
gctcacagtt tacttcgttg aaaatctgta ggggaacttt aacataaaac acttgtgaaa 1380
ttgtatagtc ctgtatgcgg tcatggcaaa ctaatgttgt catcagcggt gtttggaaac 1440
ttcacacaac ttgtaatctg ttggttcatt ctctctcctg tccatttggt atacaactgc 1500
cagacctcct tccctttcat tgtgccctgt ccatttttag aagccattca aaacctgcac 1560
ttgtataatc agtttttagt gctagaaggg gctttgccct cagtgccttc tgtgatttag 1620
tttgatcttg tctttttcac ctaatatttc tgtcccacta cacagctctg cgatcttaaa 1680
cagatctctg cttctaaagt aactttatga cagttgcagt agattctgtt tattgtggat 1740
aaaatcaagg aataaaaatt aagcggagtt aaatcttagt ggtgagatta tgggtttatt 1800
gaaacatttt tcttttgtgg aaatttatct tatgtgtgta cacatacaga catacacaaa 1860
tattttccca ttaatacttg agaatgtagg ctttgtatct aaaatctact tttattcaaa 1920
ttattcctct tgtacttgtc tttgttgatt acttaagtca tttgactcct tagccttatt 1980
gttatcatta ttaaatagac atgtagcaac ttgaaataaa actctcagtg gtagcaattc 2040
ttagttttgg taatggagca atagcctagt acagttttgt attcattgga caaattttaa 2100
tctttctgga ctttgttgat ttatataagc tgtcttatgc taaaatacat agttgtggtc 2160
cagcaagatg tcttgatgca ggaagacact tgccactaag cctgatgacc tgagttcaat 2220
acctctgacc tccatattca agctgtgtta caacctccca aatgaaacat aaagtaaatg 2280
taaagaatta aaagatatac ttatgactca agcaaggtca ttagaattca gtagttctgc 2340
ctgtgtccta gaagcccatg ttgtttccta gtctataatt ctgtaatgct acagcagcag 2400
tcactgccgc tgtgaaagtg ctttttgatg ggactgtttg ctatccttga aatgttcaag 2460
gtagtggtga gaaaatggtg agaaaactgc aaagtaggta gggaaacaat tactgactca 2520
tgagttaact atatattcag tttggccaaa gatttgcata gtagtgtcat gcaatcttca 2580
ttcttgccct gtaagggaat catcatcctc tttctataga gaaaggtatt gaaatttagt 2640

cattatcagg tgacagaacc acaattggaa taaataaatg ccttagtcca atgtttctta 2700

cactgtgtta agatttattt caatagctcc attgcttttt ttctcagtat atgaatgtat 2760

tttcagttta cttagaattt atatagacct cataataata tcacagttat gttattcatt 2820

tgctctgtaa cctgtacctt tagattaccc caatttttta tatttgtttt atgttctatg 2880

ttatcctgag ctttaaagtg accttttctt gtccatagaa tgggctgcag ttgtgagttg 2940

ctttccagaa gttctgggct aaccttgctt gaatattcta ttcttagacc catgcaggac 3000

tgtcagatgc tgccttctat ataataaagt gctttgagac atgggcttat tagtttttct 3060

tggttacagt ttacttttaa tgcataagct gtgattctcc aatctttgat tttcatgccc 3120

agtcactaaa cagtttttta aggatttacc catagcatat gcatttgtaa gttacgtatg 3180

aattttgcta gtaatttatg ttgtaaagta cttaaaactg taggttaaac tagaggaaat 3240

atgtcttcag ttatttttct attgctttga agacacaccg tgacccagac aacttataaa 3300

aaagaaagca tttaattcag agcttgttta cagtttcaga gggtgagtct aggaccatcc 3360

tggaggggaa ccaggaggca gactggcagc atggcattgg agcagtggcc gagaacatat 3420

gttttatctg caaattgggt gcagaaagta ctagagactg gggctgatgt ggacttctga 3480

gacctcaggg cctactccca gtgacaaacc tcctccaaca aggccacatt gactaatcct 3540

tcccaaagca gttccaccag ctagggacca aacattcaaa tgtaagagcc tctgggggag 3600

ggacattctc attcaaacca ccacaagatg aaaaagttga atttcatgaa acccagtgga 3660

tgctatatgt actctacctt gataactact aacttagaat aatatagagg gtgttataac 3720

tctgatgttc tggttgtttg gcagagtgct ttgagaacta tataaacagt atagaataaa 3780

tcttctgtgc agtgagtatt gtgaacatgt aaaacgctgc tttgtttctt taactagcat 3840

aaacacagga aggttgagtg aggattccgg catggtaaat cagaactata atcttagcat 3900

ttggtagatt gagacaggaa tattgctgct agtttgaggc cagctttggc tatataatga 3960

gttctaggcc agcctgggct acagaataaa aagagaatag attggaatgg gtttaagggt 4020

ttgtcgtaaa aggaaggatt agtatttttc cttagtgtag tcccccagat atgtgtacaa 4080

ccattacact agctttagtt gatgggaaaa taaaatttta attgcatctc tgttatgtta 4140

aatacatgat ttttggaggc atttgtacag attacactta gaatattaga gtcaagatac 4200

atgaaaggcc agctggttca ggtttctcag tttacatatg ggacacagat ccagagaaat 4260

ttcacatgtt gcttaagatc ttgcattcag gattaaaagc ttataaaaaa tgaacatatg 4320

ggtcaaatat tctttctaat agtatacttg tttcaagcag gatacagtat aatcaaatac 4380

aagcaagaat ttcccagagt tatatgattt tactgataat tggaaatata tctcacttca 4440

ttttctgatt ttcatttctc ttttttcttc tag 4473

<210> 19
<211> 5683

<212> DNA
<213> Cricetulus griseus

<220>
<221> Intron
<222> (1)..(1)
<223> CHO-K1 hypoxanthine phosphoribosyl transferase intron 5


<400> 19
   gtaagtgctg catttatttc taaaatactg ttaaattttc tagcttagtg tatactatag 60
   gtaagccagg gaaaaattct ttctgtttta attggggggg gggtgttttc taaagactaa 120
   gagaagccat gtacagtaag gcatgcctgg aatcccaaac actcaggaag ctcttgaatt 180
   ccatttaggt tagcctgggt tacataatag agttcggaaa ctagagagtt cttgttttta 240
   aaataaaaca aaataaaaaa aacacaaaca gaaaaggata agtgatgtat tttccagctc 300
   atgaaaaaag tttctcttat tgttttgatt ggtttctttt ctttcttggg gggatggggg 360
   gctcgaggca gggtttcttt gtggctttgg agactgtcct ggaactagct cttgtagacc 420
   aggctggtct acaagaacag agatctgcct gcctctgcct cccaagtgct gggattaaag 480
   gcctatgcca ccaatgccca gctggttttt tcttttaaaa gtagctataa ctatatagag 540
   gggtatgtgt gtgtatgtga tttactgtgc taagttttat tttatggaac atcataaggt 600
   tcttatttca tcttaatatg agtatcaaat attcttagaa aaactattta tagttcatgt 660
   gttattttcc atttgctatt atgtctttga gtactttgta tgtaatacca gtaaagttta 720
   tccagtgctt actttgccag ctactactgt aaatgatgta gaaacaagca caagaagggt 780
   aaacagattc ccaaggtcac cagagagtga gtagagtaaa gattcaaact atcactgtct 840
   gtagcctgtt gcttttcaat gtgatcccta aaaaccaaaa gcatcatcca agcaactact 900
   gactcacaat ctttatgatg aaaaccagtg agcttttctt aagcaaactt tgagctaagc 960
   tttaagaacc aatgatttac ttagaaactt tctcattaag gccttctttg actgtgctac 1020
   ataaaaatct tgtatttcca tgtcaacttc ctatcccttt cagtattttt tcacttgcca 1080
   ctgtctaaca gcatatacat atacctcttt aatagtctat ggtctcccta tttcatatct 1140
   gtctgcctta ctgaactcta tacacctgtc agtgatcatc ctcctacctt gacgctgctc 1200
   aattcttcag taaacatttt aatatatttt tagtgtggct tcaagaacct cattctccat 1260
   tatttttcat tttctctata ctttccacat tttccagaac atcgtttttc tatcattttt 1320
   ctgcccctag ttgcttactc gattttataa tctctttgtt tcctttatgc ttttttgtaa 1380
   tatttttgtg catgtgtttt gtatgtctga gtgtatatgc atgtgtatgc aagtatgtat 1440
   gcctttgcat aactacagag gccagaagag gatgtgtgtc ctttattgtg ctgtctattc 1500
   atttgagaca gggtcctctc tctgaaccag gggctcattt tattagaagc cagcaaaccc 1560
   cagcagtctt cctgcttctt gccttctcag acatgggctg caggtgggaa caagatgcct 1620
   agttttttgt ttgttttttt gatttttcga gacagggttt ctctctggct ttgaaactag 1680
   ctcttgtaga cctggtctca aggctggtct caaactcaca gagatccact tgcatgtgct 1740
   gggagtaaag gcatgtgccc accactgccc atcagatgcc tagtttttat gtgagtactg 1800
   agatccaaac tccagtgctc ctggttccca ttgagcatcc tctgcagtcc ttattttgtt 1860
   tttttttaac aaaagaaatt ttatttttta agtggggctc taggaatcaa acccagaaga 1920
   tgctaaataa atgttctacc actaggcttc accctcagtt ctttgttttg ttgtttgttt 1980
   gagaaaaagg tagctaagct aaatagtgca ggatatattc aactccatcc acctgcctct 2040
   acctcccaag tcaagtgctg gcattacagg catgtaccat catctacctc ccttctgccc 2100
   ttctctttct cattccatct ctgctctcct ccctctcctc tttctctctc ttgcctcctt 2160
   tcctctcacc cttccttcag cattatcttg gtttttgttt ttttttaaag atttatttta 2220
   tttttattat gtatacagtg ttctgtctgc atgttgactt gcacctcaga agagggaatc 2280
   agatcccatt gcagatggtt gtgagccatc atgtggttgc tgggaactga attcgggaac 2340
   tctagaagag tagccagtgc tcttaacctc tgagccatct ctccagcccc cctagaagga 2400
   acttgttgtg cagctcaaga tggctttttt cttctatgag acgaggtgtg tagccccatt 2460
   tggtctgagt ctcacagtgt ggaccaggat gacctcctct gccttcagaa tgctaccatt 2520
   acagttgggt atcaccgtac ctggcctcca agctggcttt gaacccactg tgtagcttat 2580
   gctgactttg aaccccttct gccttagctt ctcacatgat ggaattacag gtgtgcacca 2640
   ccattgttcc attagtattt ttttaatttt ttctttttcc cattttagtt ctttgagaat 2700
   ttggtatatt gcattttgat tatgttcacc tccactccac tccccccaaa tctgctccca 2760
   cttccctatt tacccaacat tgtatcttct tcttttcttt aaatccaaca atacagttag 2820
   tgctgtacat tattcctgga tgtgtggcct tcactagaga gtggtcaccc cactaggagc 2880
   cacattgtta aagaaaactg actcctcttt gcaacaccta ccaattgcta tttgcaatta 2940
   aaggtggtgg tagtggcaca cgcctttaaa cccagcactc gggaggcagg ggcaggtaga 3000
   tttctgtgag tttgaggtca gcctggtcta cagagcgagt tccaggacag cctccaaagc 3060
   cacagagaaa ccctgtccca aaaagccaaa agaaattgat ccttcactag aggtaggaca 3120
   tgatgccctc ttccccctcc atgctggaat tttgtctggc ctggccctaa acaggttttg 3180
   taatgctttc tcaactgata tgaactcata agtgcaactg cagtactgtg tcagaaaaca 3240
   ctgtttcctt gaagttatct acgactttgt ggctcttttt ctttaattaa aaaagtttgt 3300
   tgccaggtga tggtggtaca cacctttaat cccagcacta gggaggcaga cacagaggca 3360
   gaggcaggca gatatctgtg actacagaca gagttccagg acagaaaaac ccacacagag 3420
   aaactctatc ttgaaaaaaa aaatagtttg tatttgtttg catgtgtaaa catgccacag 3480
   tatgcacatg gaggccagaa gacaactttg aaattgcttc ttctaccttt tttaaaaagt 3540
   ttaaattaga aacaagtttc tttacatgtc aatcctatgc tggtttccca gctatcagtc 3600
   tggggtccat aagcaacccc ttctaccttt tgcatggaat ttggggattg aatttaggtc 3660
   actgggctta tatggagaat ggctttattc accaatccat cttggttgcc cttaattgtt 3720
   atttttaaga gatttattta tttataatgt acaatgttct gcctacatgt ctccctgccg 3780
   gccacaagag ggcaccagat ctcattatag atggtggtga gccatcatgt ggttactggg 3840
   tatgaactca ggatctcagg aagagtagcc agtgctctta acctctgagc catctctcca 3900
   accaccttac ttttttcagt aatattataa aaacatcttc atctcgctac gcagaaatca 3960
   gtccccttag cctttattta tttatttatt tatttatttg tttgtttatt ttttggtttt 4020
   tcgagacagg gtttctctgt ggctttggag gctgtcctag aactagctct tgtagaccag 4080
   gctggtctca aactcagaga tttgactgcc tctgcctctc gagtgctggg tttaaaggtg 4140
   tacactgcca ccacccggct agccttcatt ttttatttct tccttcccct tctctacttt 4200
   ctaagatttg cctattgagg acatagcaca aatggaatta catagtaatt aattgtactt 4260
   tgtgtctggc ttttttcaat tattttaaga tagggtctca ccatgtggcc ctggctgatt 4320
   agctatgtaa tcgaggctgg ctttgaacca cacaccccac aaataaataa acattcactc 4380
   aggatgatag cactgtatag ttacagtaat tatccaaatg aggcatagtt ttctctctgg 4440
   ctgttggcca gggattgctc tcatttacag agatcatccc tcatgtccta cacagcctct 4500
   catagcatgc ctgctcactt cttccactct gtactctagg gggcggtaat ttttgtgcca 4560
   tcagtattct gcctgccaca ccctagaatc ttggtttttc acaagtctct agccctgaat 4620
   ttctaatgtc cacattgtct tacgtatttt tttttctttt ggttttttga gaaagggttt 4680
   ctctgtgtag cttttggagc ctatcctggc actctctgga gaccaggctg gcctcgaact 4740
   catagagaac cgcctgcctc tgcctcccga gtgctgggac taaaggcatg caccaccaac 4800
   gcctggcttt gtcttacgga tttttatttg ttttcttttt cttttctttc ttgttgtttg 4860
   ttttttgttt gtttttgttt ttgagacagg atttctctgt attattttgg aacctgtcct 4920
   ggaactcact ctgtatacca ggctggcctc gaactcacag agatccacct gcctctgctt 4980
   cccgagtgct gggattaaag gtgtgcacta ccaacgcccg actttttatt tgttttctga 5040
   taccagtttt ggtaatactg gagatctgcg gctcagtgga tcttatgcag gtcttttacc 5100
   cctgataaag tcattttata tagtgaaact tagccatttg aatgccttca aaaataatac 5160
   aaagcaccat ttctcacagt attagagttt gagatgaaag agtccctatc ttgaagttat 5220
   ggagaaaaat ctaaaatact tttacagaat taagcatagt ggcttatgtc tgccatccta 5280
   gctgtcagga gtctaaatag gaggctcttg agtttaagga cactagcctg agctatttag 5340
   ctagacccgg tgtggaaaaa caacaagaag atttacatgt atgtacattg cttacatgta 5400
   cccatttcaa ttcattgttt ttgacctcac tatacctatg cagcctagtt ctgggaactg 5460
   acattcccac tacttgctga aagccagatg ggttgcaagt tctagaatct ctgtgtgtga 5520
   attgtgagaa tttttttatg tgctgaaaaa taatgacatc gtactgtgga ataccagaac 5580
   actgggattc tgtatattct gtgtttagat agtgactctg attttaaaat tatgctggtt 5640
   ttacttacca cttaccatta aatacctctt ttctttttaa aag 5683

<210> 20
<211> 4534
<212> DNA
<213> Cricetulus griseus

<220>
<221> Intron
<222> (1)..(4534)
<223> CHO-K1 hypoxanthine phosphoribosyl transferase intron 6

<400> 20
gtatgtatgc cattttaaag tagaatgttt ttttttcatt gggagcaata agcaattgct 60

tctttgtaag gagaaatgct ttcagctctc tttttatctt gaaaaggtga tgtaatctta 120

aaatttagta tactttgtta ttaaaaccct tcattatata gtcaactact tgtctaatac 180

agtatataca gggacttgga ttcagaacca cctcagacac taagctccct agatgttcaa 240

gtcctttgta aaaaaaaaaa aagtcagatg ggctggcaag aggactcagt gggtgaagtc 300

acctgctgcc aaccctgatg acctgagttc aaaccctggg acccacatgg tagaaggaga 360

gaacggactc ctagaaattt ccctgtgacg tgtgtatcac aacacatgtc ccccaaccca 420

cagacaagta aatgtaattt aattttttta aatgtcaaga gtgttttcac agaacctatg 480

catattctcc cttatacttt aatacctatt gcaatgtcat ataaataatt atgctgaatc 540

atctaaggaa atatgtatag ttttagtata gtttcttaga ctatttgtgt gtgtgtgtgt 600

gtgtgtgtgt gtgtgtgtgt gtgtacatgc aggtgaatgc acacaggcac atatgcaaag 660

gccagagcag gattggatat cttcctctat tgtgtaatca gggtctctaa ctgaggtata 720

agtttaaagt tttagccatg caggagtttg gtgggtgagc ttttgaatcc ctctctaacc 780

tcacagtact ggagtcacaa acacatacag ccatggccag atttttatgt gaatactgaa 840

gaggcaaact caggtacctg tgctttagga ccaacttgat acacaaggtt tagatacaga 900

actctcacac atatgtagag tcaagtaaca attggttttg ccacttgcta tttatatcag 960

agttgtcctg agtagcatgc aaggatggat ctaggagccc ccttgtgaaa cagaaaccgg 1020

cgatacccat agggttgggc tctgaacatg tggatctcct gaagttgagt ggtggataag 1080

gcatcctgtt ttgtgtcatc attgacattt tacaatacct gctttttttc tgaagcagtc 1140

acatatcttc ctggccgtat ctagatgttt ggctttgctt tcatagttct ttctgttttt 1200

atccttagtt acatagtttg atcctacaac agttgttatc gccatcttac agatgagaat 1260

gttgaagcgt agggaattgc ccacagacag agctaataag ctgtagagag atgatttgaa 1320

tacagggtac ctggctcagt tgaggcttct gttattttaa gttggggttt ttgggttttt 1380

tgcttttgtt tttcgagaca gtgtttctct gtggctttat caggctggtc tcgaactcac 1440

agagatccgc ctgcctctct gcctcctaag tgctgggatt aaaggtgtgc tacaccaatg 1500
cccagtttaa gttgtttttt tttttattgt tagaagtaac attgttttgt tgttgttgaa 1560
ttttgctttg tttttacttt acaatttcat ttttattttc aaatctttga ctaatctgtg 1620
ttttttgtgt agaggaaagt tcgggtctgt ttttcttttt tccctgaatg attaactggt 1680
tcttgacatt ttttgaatta cacttattta ttgtggcagg gatgtgggca tgtgccacat 1740
gcacttgtgt aggtcagagg atagcctggg ggagttaggt ctctaacttg ttggtcgtag 1800
gtgggggtag aatacagctt ggtagacttg gcagcaattt ccttacccac tgaaccatct 1860
ctgctgacca attagctgcc ttttataaac cattttaaag gatcaaagta tcttgtaaag 1920
tctttgaata tcattttaat aaagcctatt tgagcagttc atcctcaagg actcttctga 1980
tagatggtgc aagtgtacct cagtgagttt ttgtcttgct ggaggtgcta tatgctcaag 2040
agaatgggta atttttgata atgtagttgt cctatacagg tgtgttttgt gtttttccca 2100
aaataaaaat tgaaaagcta ttatttgata tgaatatatg atagtatggc cactctagaa 2160
aacaattttt ttttgaaaac tgtacatgta actaccagaa ggccttacaa attatactgg 2220
gtacttatac ctgagaagta aaagtgcaca ttgatataaa aatttatata agttgatagc 2280
taggtatggt ggttcacccg tgacgtcagc atttggaaag tggaggcagg aggtcacatt 2340
gaatctaaga caagcagcct gagttttcat agctgtctcg tagcttccta ctcaaatctg 2400
taccagagta ttcacaacac catttgtgat agcccacaat taaaagcgac aaactgaaca 2460
aaactgtagt acacctatac catcatattc atcagtaaac aggaacaact attgattgat 2520
atacaacagt tcagagaagt ctaagtggaa ctgtaccaat tatgactttc agtagtacat 2580
aaagcaggtg ttttgctctg tttaaactgc ttacctgccc cattttcccc caagactatt 2640
ataagccccc ctcaaagaaa agtaataaaa ctgtatgatt ctacttaaat tacctacttt 2700
gaaatggcaa agtcatacag atgagaacag attaggggtt gccaggggtt aagggaatgc 2760
agaaggagcc cttgcatgtt tcaaatatct tgattatggt agtgattaga taattccaca 2820
tgtgactaaa attgcctgaa ccgcacaaaa agagatgagt ttatataaaa ttggtgaaat 2880
ctgtacatgg tcacttagtt catcaatgcc catgtcctgg tttgataata tgctgaaaaa 2940
ggacactgga gacctccctt ttgtatgatt tttttcattt gcaacttcct gtgaaaataa 3000
gaaacattga aagccattaa ctattttggt ggtattaggg attcaactca gggttgtatg 3060
catacatgac aagcattcag ataattagct ttagagctgt atgtaattaa ttctgtaatc 3120
aaattacagt tgcaaattca tgatttctat aagtactcaa taacagcaaa gtaattttaa 3180
aaaatgaaac tgctcaaagc tattatgttt ttagcaagag tcaaacagtt gaaaatataa 3240
ttaactccaa atgatgtgag ggacgttgat ggtcccccgt ggagggcttc actatccatg 3300
gatgggggtg ggttggggga ggtcggcaga gagcatagga ggatgggagg gtgagggaat 3360

gggggactga tatgtaaata agagtgcttc taaaattaaa aaaggaaaat ataattaact 3420

aaaaaatgga gtaggtagct cagtgataat atacatcctt tttttgctgg ggggaggggg 3480

ggatgagttc aagatggggt ttctctgtgg cttttgtggc tgtcctgcaa ctagcttttg 3540

tagaccaagc tggtctcgaa ctcacagaga tccgcctgcc tctgcctccc aagtgctggg 3600

attaaaggtg tgtgccacca gtgcccagct ggtaaaatcc attcttaaca tgatcaagtc 3660

tgtgggttca atctccacag caaaaaataa tttaaaaaat ggttcttttg gggctggaga 3720

gatggctctg cggttaagag cactgactgt tcttccagag gtcctgagtt caattcccag 3780

caaccacata gtggctcaca accacgttga atgagatcta gtgccctctg ctggcatgca 3840

ggcagaagac tgtatacaaa ataaataaat aaataaataa ataaataaat aaataaataa 3900

gtaaataaat aagtaagtaa ataaataaga attattttaa aaaatggttc tttttttgga 3960

gacagggttt ctctgtagcg ttggagccta tcctggcact ccctctggag accaggctgg 4020

cctcgaactc agagatccgc ctgcctctgc ctcccgagtg ctgggattaa aggcatgcgc 4080

caccaacttt taacacactt gttttgtagc ctaaagttcc catttgtgag gcagttatta 4140

agagtcttga tgaaagagca ttttttaccc acctccttta taagttgagg ggttgtattt 4200

caaggattct gaatctagat ccccttattt tgaatctttg tgtgttggta ccttggccca 4260

ttcattattc tcagtagctg cacttcaaag gacatatggg tagttaggat tccactctca 4320

gaagaagcat cttatcaggt tccaggtgga gatcagtcag gggccttgtc ttgttgtaaa 4380

ctctgatgtg ggtctccatt tgtcttccta tgggtatgca aaactgacct tattctaaat 4440

gcagatgata ctatttaaga acactgatga tgttttcttt tataatgttc tattgtcttt 4500

ccatatgtca catgtaatat tttgtaatta acag 4534

<210> 21
<211> 166
<212> DNA
<213> Cricetulus griseus

<220>
<221> Intron
<222> (1)..(166)
<223> CHO-K1 hypoxanthine phosphoribosyl transferase intron 7

<400> 21
gtaagtagat gccctttggg tgatcatttt atctatcata gctgaaaggt gactaaaatt 60

gcttagagtt attttagaga acgtttgtaa agaaggcaca tttgcaaata ttagatttga 120

aagtactgtt ttctctgtct aaataataaa ctgtgattct ttacag 166

<210> 22
<211> 976
<212> DNA
<213> Cricetulus griseus

<220>
<221> Intron
<222> (1)..(976)
<223> CHO-K1 hypoxanthine phosphoribosyl transferase intron 8


<400> 22
gtaagtaatg tttctttttt tctcgctctc atttttcaaa aaacccacat aaaaatttag      60

gaaaggaaag aattgttttc tccttctagc acctcgtcat ttgaccagac tgatggttcc     120

cattagtcac ataaagctgt agtcaagtac agacgtcctt agaactggaa cttggccagg     180

ctagggtgac acttcttgct ggctgaaata gttgaacagc cttaatttac aataactgcc     240

tcattgttat ttcacatgat aaatatagtc ataaataaga aataaataat tccatttaaa     300

tcttatgatt gaccctactt cagatacctg cctcccactc tggaggcaag aatccctcag     360

gtgagtttat gaacatggtc caggagaaat taagaagact acgtggacag cattatctaa     420

agcagttttg tttgtttgat tggttttcaa gatagagttt ctctgtttaa ccactctggt     480

tgtcctggaa ctcaatttgt agaccagacc acagagatct gcctgcctct gcctctcaag     540

tgctgggatt aaaggcccaa agtacagttt ttatttcatt tttttaatca cttaccctat     600

tttctttgag acaggattta tcttgtagta ctggctgtcc tgaaactcac tctgtagacc     660

agattggcct cgaactcaca gagaatccat ctctctctct attgggatta aaggcttttg     720

ccatcatcac ctagccacac tgatccctat tttgatatgc cttcaatgcc cggctttata     780

tgtttttcaa agttacataa taagagaaag aaaaaagtac actaatggaa catatttctg     840

ctttgttttc aaaatgtgtt ctcttcaagt tactggtcca aagtaccttg tttggtagga     900

acccagacaa ttctctaatg ttgctcttac ctctccagaa tatatttttc taatgtgagt     960

ttcttttctt ttgcag                                                     976


<210> 23
<211> 40
<212> DNA
<213> Artificial

<220>
<223> Designed oligonucleotide primer to amplify a portion of hprt sequence
from HT1080

<400> 23
cctgcaggtc gcgattggta cttgttcagc tttattcaag                            40


<210> 24
<211> 41
<212> DNA
<213> Artificial

<220>
<223> Designed oligonucleotide primer to amplify a portion of hprt sequence
from HT1080

<400> 24

gtcgacaagg acgcgttctg ataaaatcta cagtcatagg a                41

<210> 25
<211> 67
<212> DNA
<213> Artificial

<220>
<223> Designed oligonucleotide primer to amplify a portion of hprt sequence
from HT1080

<400> 25
gtcgacctct agctagagct tggcgtaatc atggtctccg gagactgaag agctattgtg     60

tgagtat                                                               67

<210> 26
<211> 46
<212> DNA
<213> Artificial

<220>
<223> Designed oligonucleotide primer to amplify a portion of hprt sequence
from HT1080

<400> 26
acatgttctc ttaagtcgcg aagtagtgtt atgatgtatg ggcata                    46

<210> 27
<211> 35
<212> DNA
<213> Artificial

<220>
<223> Designed oligonucleotide primer to amplify the E.coli origin and
ampicillin resistance gene

<400> 27
acatgtgagc aaaaggccag caaaaggcca ggaac                                 35

<210> 28
<211> 40
<212> DNA
<213> Artificial

<220>
<223> Designed oligonucleotide primer to amplify the E.coli origin and
ampicillin resistance gene

<400> 28
cctgcaggga cgtcaggtgg cacttttcgg ggaaatgtgc                            40

<210> 29
<211> 36
<212> DNA
<213> Artificial

<220>
<223> Designed oligonucleotide to make a linker

<400> 29
cgcgtatctc tagaataatc gatagaaagc ttacag                                36

<210> 30
<211> 36
<212> DNA
<213> Artificial

<220>
<223> Designed oligonucleotide to make a linker

<400> 30
gatcctgtaa gctttctatc gattattcta gagata                                36

<210> 31
<211> 30
<212> DNA
<213> Artificial

<220>
<223> Designed oligonucleotide primer to amplify the SV40 promoter, SV40
polyA signal and neomycin resistance gene

<400> 31
cctttctaga cttctgaggc ggaaagaacc                                       30

<210> 32
<211> 79
<212> DNA
<213> Artificial

<220>
<223> Designed oligonucleotide primer to amplify the SV40 promoter, SV40
polyA signal and neomycin resistance gene

<400> 32
cttatcgatt cacacaaaaa accaacacac agatgtaatg aaaataaaga tattttattg     60

tgggcgaaga actccagca                                                   79

<210> 33
<211> 30
<212> DNA
<213> Artificial

<220>
<223> Designed oligonucleotide primer to amplify an antibody variable region

<400> 33
taaaaggtgt ccaggatgtg cagtttcagg                                       30

<210> 34
<211> 30
<212> DNA
<213> Artificial

<220>
<223> Designed oligonucleotide primer to amplify an antibody variable region

<400> 34

gaggccgatg aaacagtgac cagagtccct                                        30

<210> 35
<211> 30
<212> DNA
<213> Artificial

<220>
<223> Designed oligonucleotide primer to amplify an antibody constant region

<400> 35
catcggcctc caccaagggc ccatcggtct                                        30

<210> 36
<211> 30
<212> DNA
<213> Artificial

<220>
<223> Designed oligonucleotide primer to amplify an antibody constant region

<400> 36
ttaagcggcc gctcatttac ccggagacag                                        30

<210> 37
<211> 70
<212> DNA
<213> Artificial

<220>
<223> Designed oligonucleotide primer to amplify an antibody constant region

<400> 37
ggtcgccacc atggagtttg gactgagctg ggttttcctt gttgctattt taaaaggtgt      60

ccaggatgtg                                                              70

<210> 38
<211> 30
<212> DNA
<213> Artificial

<220>
<223> Designed oligonucleotide primer to amplify an antibody variable region

<400> 38
gcccttggtg gaggccgatg aaacagtgac                                        30

<210> 39
<211> 30
<212> DNA
<213> Artificial

<220>
<223> Designed oligonucleotide primer to amplify an antibody variable region

<400> 39
ttccggtacc ggtcgccacc atggagtttg                                        30

<210> 40
<211> 28
<212> DNA
<213> Artificial

<220>
<223> Designed oligonucleotide primer to insert a NotI site into the
upstream of CMV promoter

<400> 40
atgcggccgc atgtcaatat tggccatt                                          28


<210> 41
<211> 29
<212> DNA
<213> Artificial

<220>
<223> Designed oligonucleotide primer to insert a NotI site into the
upstream of CMV promoter

<400> 41
gacatgcggc cgcatgggag gagaccggg                                         29


<210> 42
<211> 30
<212> DNA
<213> Artificial

<220>
<223> Designed oligonucleotide primer to amplify a spacer

<400> 42
ttgcggccgc gaaaaagcct gaactcaccg                                        30


<210> 43
<211> 30
<212> DNA
<213> Artificial

<220>
<223> Designed oligonucleotide primer to amplify a spacer

<400> 43
ttgcggccgc gacggtgtcg tccatcacag                                        30


<210> 44
<211> 60
<212> DNA
<213> Artificial

<220>
<223> Designed oligonucleotide primer to amplify IGH

<400> 44
ccttggtacc gaagccgcta gcgctaccgg tcgccaccat ggagtttgga ctgagctggg        60


<210> 45
<211> 30
<212> DNA

<213> Artificial

<220>
<223> Designed oligonucleotide primer to amplify IGH

<400> 45
ccttagatct tcatttaccc ggagacaggg                                        30


<210> 46
<211> 30
<212> DNA
<213> Artificial

<220>
<223> Designed oligonucleotide primer to amplify IGH

<400> 46
ccttacgcgt gacggtgtcg tccatcacag                                        30


<210> 47
<211> 30
<212> DNA
<213> Artificial

<220>
<223> Designed oligonucleotide primer to amplify IGH

<400> 47
cttacgcgta acgtctcgcc ctttggtctc                                        30


<210> 48
<211> 30
<212> DNA
<213> Artificial

<220>
<223> Designed oligonucleotide primer to amplify an antibody variable region

<400> 48
cccaggtgcc agatgtgaca tcaagatgac                                         30


<210> 49
<211> 30
<212> DNA
<213> Artificial

<220>
<223> Designed oligonucleotide primer to amplify an antibody variable region

<400> 49
gccacagttc gttttatttc caactttgtc                                         30


<210> 50
<211> 30
<212> DNA
<213> Artificial

<220>
<223> Designed oligonucleotide primer to amplify an antibody constant egion

<400> 50
tggaaataaa acgaactgtg gctgcaccat                                        30


<210> 51
<211> 29
<212> DNA
<213> Artificial

<220>
<223> Designed oligonucleotide primer to amplify an antibody constant region

<400> 51
ttaagcggcc gcctaacact ctcccctgt                                         29


<210> 52
<211> 80
<212> DNA
<213> Artificial

<220>
<223> Designed oligonucleotide primer to amplify an antibody constant region
with secretion signal

<400> 52
ggtcgccacc atggacatga gggtccccgc tcagctcctg gggctcctgc tgctctggct      60

cccaggtgcc agatgtgaca                                                  80


<210> 53
<211> 30
<212> DNA
<213> Artificial

<220>
<223> Designed oligonucleotide primer to amplify an antibody variable region

<400> 53
gccacagttc gttttatttc caactttgtc                                       30


<210> 54
<211> 30
<212> DNA
<213> Artificial

<220>
<223> Designed oligonucleotide primer to amplify an antibody variable region

<400> 54
ccttggtacc ggtcgccacc atggacatga                                       30


<210> 55
<211> 30
<212> DNA
<213> Artificial

<220>
<223> Designed oligonucleotide primer to amplify a spacer

<400> 55
ttgcggccgc ctgtgatgga cgacaccgtc                                       30

<210> 56
<211> 30
<212> DNA
<213> Artificial

<220>
<223> Designed oligonucleotide primer to amplify a spacer

<400> 56
ttgcggccgc ctattccttt gccctcggac                                        30


<210> 57
<211> 58
<212> DNA
<213> Artificial

<220>
<223> Designed oligonucleotide primer to amplify IGL

<400> 57
ccttggtacc gaagccgcta gcgctaccgg tcgccaccat ggacatgagg gtccccgc        58


<210> 58
<211> 30
<212> DNA
<213> Artificial

<220>
<223> Designed oligonucleotide primer to amplify IGL

<400> 58
ccttagatct ctaacactct cccctgttga                                        30


<210> 59
<211> 30
<212> DNA
<213> Artificial

<220>
<223> Designed oligonucleotide primer to amplify IGL

<400> 59
ccttaagctt ctattccttt gccctcggac                                        30


<210> 60
<211> 30
<212> DNA
<213> Artificial

<220>
<223> Designed oligonucleotide primer to amplify IGH

<400> 60
cttaagctta acgtctcgcc ctttggtctc                                        30


<210> 61
<211> 38
<212> DNA

<213> Artificial

<220>
<223> Designed oligonucleotide primer to amplify a portion of hprt sequence

<400> 61
ttcctgcagg tcgcgaagga gtttattaga ggaaatat                              38


<210> 62
<211> 30
<212> DNA
<213> Artificial

<220>
<223> Designed oligonucleotide primer to amplify a portion of hprt sequence

<400> 62
ttacgcgttg ataaaatcta cagtcatggg                                       30


<210> 63
<211> 28
<212> DNA
<213> Artificial

<220>
<223> Designed oligonucleotide primer to amplify a portion of hprt sequence

<400> 63
ttggatccga ctgaagagct actgtgta                                         28


<210> 64
<211> 34
<212> DNA
<213> Artificial

<220>
<223> Designed oligonucleotide primer to amplify a portion of hprt sequence

<400> 64
ttacatgttc gcgaatcaga tccctgggac tgga                                  34


<210> 65
<211> 20
<212> DNA
<213> Artificial

<220>
<223> Designed oligonucleotide primer to amplify a portion of hprt sequence

<400> 65
tgttcctgtg catactaggc                                                  20


<210> 66
<211> 24
<212> DNA
<213> Artificial

<220>
<223> Designed oligonucleotide primer to amplify a portion of hprt sequence

<400> 66
ccagagaaat tatttgccac cagc                                              24

<210> 67
<211> 41
<212> DNA
<213> Artificial

<220>
<223> Designed oligonucleotide primer to amplify a portion of hprt sequence

<400> 67
ttcctgcagg tcgcgagtct gtgtgtatgt ttgtgatagg c                           41


<210> 68
<211> 39
<212> DNA
<213> Artificial

<220>
<223> Designed oligonucleotide primer to amplify a portion of hprt sequence

<400> 68
ttccatggtc gcgatgaagg ttatagagca taggggacc                              39


<210> 69
<211> 20
<212> DNA
<213> Artificial

<220>
<223> Designed oligonucleotide primer to amplify a portion of hprt sequence

<400> 69
gacacatgca gacagaacag                                                   20


<210> 70
<211> 20
<212> DNA
<213> Artificial

<220>
<223> Designed oligonucleotide primer to amplify a portion of hprt sequence

<400> 70
gtttgctaac accccttctc                                                  20


<210> 71
<211> 28
<212> DNA
<213> Artificial

<400> 71
agaacgcgtc cacacaatca gaaccaca                                         28


<210> 72
<211> 27
<212> DNA
<213> Artificial

<400> 72
gacgatctag aggtggtttt cacaaca                                          27


<210> 73
<211> 20
<212> DNA
<213> Artificial

<400> 73
agggactttc cattgacgtc                                                  20


<210> 74
<211> 30
<212> DNA
<213> Artificial

<400> 74
cttacgcgta acgtctcgcc ctttggtctc                                       30


<210> 75
<211> 28
<212> DNA
<213> Artificial

<400> 75
agaaagcttc cacacaatca gaaccaca                                         28


<210> 76
<211> 27
<212> DNA
<213> Artificial

<400> 76
gacgaattcg cggtggtttt cacaaca                                          27


<210> 77
<211> 26
<212> DNA
<213> Artificial

<400> 77
tatgaattcg tcgccaccat ggacat                                           26


<210> 78
<211> 30
<212> DNA
<213> Artificial

<400> 78
tgtaagcttt accacatttg tagaggtttt                                       30


<210> 79
<211> 28
<212> DNA
<213> Artificial

<400> 79
tttctagata gttatgagcc catgtccc                                         28

<210> 80
<211> 28
<212> DNA
<213> Artificial

<400> 80
tttctagacg gtgaaatcct gtctctac                                          28


<210> 81
<211> 30
<212> DNA
<213> Artificial

<400> 81
ttggcgcgcc tagttatgag cccatgtccc                                        30


<210> 82
<211> 28
<212> DNA
<213> Artificial

<400> 82
ttacgcgtcg gtgaaatcct gtctctac                                          28


<210> 83
<211> 28
<212> DNA
<213> Artificial

<400> 83
ttagatctta gttatgagcc catgtccc                                          28


<210> 84
<211> 28
<212> DNA
<213> Artificial

<400> 84
ttagatctcg gtgaaatcct gtctctac                                          28


<210> 85
<211> 38
<212> DNA
<213> Artificial

<400> 85
ttcctgcagg tcgcgatgga atcttctatt cctgattt                               38


<210> 86
<211> 34
<212> DNA
<213> Artificial

<400> 86
ttacatgttc gcgatcagca ctcaggagtc agag                                   34

<210> 87
<211> 36
<212> DNA
<213> Artificial

<400> 87
ttcctgcagg tcgcgagatg cctagcatgt acctgg 36

<210> 88
<211> 39
<212> DNA
<213> Artificial

<400> 88
ttacatgttc gcgataagta caaatccatc ttgggtgac 39

<210> 89
<211> 40
<212> DNA
<213> Artificial

<400> 89
ttatgcattc gcgaatctca ggtgatagga gacataagac 40

<210> 90
<211> 28
<212> DNA
<213> Artificial

<400> 90
tttgatcaga ctgaagagct actgtgta 28

<210> 91
<211> 36
<212> DNA
<213> Artificial

<400> 91
tttcatgaat cgcgaatcag cactcaggag tcagag 36

## Description

### [Field of Invention]

The present invention relates to a DNA construct useful for efficient generation of recombinant CHO cells and a process for generating recombinant CHO cells using the same.

### [Background Art]

Various recombinant protein production systems using procaryotes or eucaryotes as host cells are known. In recombinant protein production systems using mammal cells as host cells, proteins derived from higher animals including humans can be subjected to post-translational modifications such as glycosylation, folding, and phosphorylation in a similar manner to those produced in vivo,

The post-translational modification is necessary for the reproduction of physiological activities of native proteins by recombinant proteins, and protein production systems using mammal cells as host are commonly used in recombinant protein production systems used in medicaments that are particularly required to have such physiological activities.

At the present time, two protein production systems, CHO-DHFR systems and GS-NS0 systems, may be mentioned as main protein production systems using mammal cells that are used in productions on a commercial scale. In these production systems, clones that can realize increased copy numbers of a plasmid vector integrated into chromosomes are screened by combining a selective marker contained in the plasmid vector with a proper drug selection process. In particular, in CHO-DHFR systems, cell clones, of which the expression level has been increased by a factor of several tens, can be screened by a two-stage screening process using a selective drug Methotrexate.

However, it is known that the above protein production systems suffer from problems, for example, that the expression level of the target protein is not simply always increased proportionally with the number of copies of the amplified plasmid vector and that a lot of time is taken for screening of cell clones having an increased expression level. Further, it is reported that, when, after screening of cell clones having an increased expression level, the culture of selected cell clones is continued in a medium free from a selective drug, a reduction or disappearance of the expression level is observed in most of the clones (PTL 1: JP 2002-541854T, NPL 1: Kim, N. S. (1998) Biotechnol. Bioeng., 60, 679-688).

Further, in target protein production systems using mammal cells, in general, a target protein is produced by introducing a vector containing a target protein gene into host cells, screening cell clones in which the vector has been integrated into chromosomes, and further culturing the cell clones under proper culture conditions.

The integration into chromosomes often occurs at random positions, and the target protein expression level varies depending upon the cell clones obtained. Further, some cell clones suffer from problems, for example, that they do not express the target protein. To overcome this drawback, a method is adopted in which a number of clones are selected according to a recombinant protein expression level, and favorable clones are selected. This screen process, however, is very troublesome and takes a lot of work. Various processes have been reported for avoiding such a troublesome task and quickly selecting favorable clones.

For example, a technique is disclosed in which a vector is integrated into a specific chromosomal position of mouse cells (PTL 2: JP 9(1997)-510865A). Homologous recombinant cell clones are produced in a recombinant cell clone pool by a vector loaded with a sequence having a base sequence homologous to immunoglobulin y2A locus. A target chromosomal position is previously identified as a position that, when a foreign gene is integrated, can provide a higher expression level than random integration. Accordingly, when homologous recombinant cell clones having a high expression level are present with given frequency in a recombinant cell clone pool as a screening object, work in screening according to the expression level can be reduced.

A technique for the utilization of a marking plasmid is also disclosed (PTL 3: JP 2001-516221A). Clones having a high expression level of a marker gene present within a marking plasmid are previously selected from a cell clone population obtained by random recombination of the marking plasmid. Next, target protein producing clones that have inherited an expression level of the marker gene are obtained by selecting cell clones in which site-specific recombination has occurred between the plasmid vector having the target protein gene and the randomly integrated marking plasmid sequence.

The above technique is advantageous in reducing work necessary for selecting clones having a high expression level. However, for example, when recombinant cells are continuously cultured without the addition of a selective drug, it is unpredictable whether or not the clones thus obtained can stably maintain the expression level for a long period of time.

In the production of medicinal proteins on a commercial scale, it is important that the expression of proteins is stably maintained at a high level. In particular, stably maintaining the expression level is important from the viewpoint of cost, as well as from the viewpoint of proving identity and safety as medicinal proteins. In order to use recombinant protein producing cells in production on a commercial scale, it is necessary to increase a culture scale of producing cell clones. It is estimated that, in general, at least about 60 times of cell divisions are necessary from clones immediately after the establishment (NPL 2:Brown, M. E. et al. (1992) Cytotechnology, 9, 231-236), and the expression level should be maintained at a constant level.

Further, selective drugs incur an increased culture cost and, at the same time, incurs an increase in cost involved in a purification process provided to avoid a possibility of mixing foreign matter into medicaments. Accordingly, the development of a technique for manufacturing of cell clones that can stably maintain the expression level without the addition of a selective drug has been strongly desired.

Despite the above circumstances, it cannot be said that satisfactory technical studies have been made on the stability of the target protein expression level. Up to now, in many processes for generating protein producing system, the selection of clones has been empirically made based on accumulated data on growth rate and productivity in long-term culture. This empirical clone selection method, however, can hardly realize the acquisition of cell clones that have a stable expression level (NPL 3:Barnes, L. M. et al. (2003) Biotechnology and Bioengineering, 81, 631-639).

A technique has recently been studied in which a target protein gene is specifically integrated into a target gene locus in a cell genome to acquire efficiently recombinant cells that can express a protein for a long period of time in a stable manner.

An hprt gene may be mentioned as one example of the target gene. The hprt gene is known as one of housekeeping genes located on the long arm of X chromosome, for example, in humans. Cells after knockout of the hprt gene are resistant to drugs 6-Thioguanime (6TG) and G418, and, thus, negative selection is easy.

Some of the present inventors have reported that recombinant cells that can stably express proteins in the absence of a selective drug for a long period of time have been acquired by introducing a target protein gene into an hprt locus of a human male-derived HT1080 cell line through a recombinant vector (PTL 5: JP 2007-325571A, NPL 4: Koyama Y Et Al., (2006) Biotechnology And Bioengineering, 95, 1052-1060). It is reported in an experiment of this document that about 10 clones of recombinant cells per 10⁷ cells were acquired by using an about 1-kbp first homologous DNA fragment and an about 1-kbp second homologous DNA fragment as homology arms.

Further, targeting to mouse ES cells is reported as another example of targeting to the hprt locus (PTL 6: JP 5(1993)-507853A).

However, even when the target is an identical locus, a gene targeting frequency sometimes significantly varies depending upon the type of culture cells. For example, in Porter C. G. Itzhaki J. E, Eur. J. Biochem 218, 273-281 (NPL 5) and Annual Report of the Hiroshima University Research Institute for Radiation Biology and Medicine, vol. 44 (2003) (NPL 6), it is reported that ES cells and HT1080 cells are different from each other in gene targeting frequency and, in somatic cell-derived culture cells, the gene targeting frequency is very low.

On the other hand, CHO cells are utilized as host cells in antibody medicinal protein producing systems, and the construction of a high-level and stable protein producing system using CHO cells have been demanded.

However, there is no report that gene targeting has been done to hprt locus of CHO cells. There are only studies on the use of special cell lines that are deficient in an aprt gene on one chromosome of CHO cells (NPL 11: PNAS, 88, 9488-9502 (1991), NPL 12: Somatic Cell. Mol. Genet., 19, 363-375, NPL 13: PNAS, 86, 4574-4578 (1989)). In these experiments, a cell line in which an aprt gene is present only on one chromosome is used as a host, and 2.6 kbp to 4 kbp-homologous DNA fragments are used as homology arms. As a result, homologous recombinants of approximately several clones to 15 clones per 10⁷ cells are acquired.

There is no report on sequences of genome of an hprt locus of CHO cells except for exons. Accordingly, specific gene targeting to an hprt locus of CHO cells poses a problem that, at the outset, all base sequences other than exons should be analyzed and identified.

Further, CHO cells are female-derived cells and thus have two X chromosomes that have two hprt locuses. Accordingly, when a foreign gene is integrated into both hprt locuses of the chromosomes, CHO cells become resistant to selective drugs such as 6TG. The probability of recombination in such two chromosomes is generally lower than that in male-derived cells. For example, when the probability of recombination in one chromosome is presumed to be 10⁻⁶ while using the efficiency of recombination into an hprt locus of a male-derived HT1080 cell line in Koyama Y Et Al., (2006) Biotechnology And Bioengineering, 95, 1052-1060 (NPL 4) as a reference, the theoretical probability of simultaneous recombination in two chromosomes is 10⁻¹².

Further, in female-derived cells, one of two X chromosomes is inherited paternally and the other is inherited maternally. Accordingly, polymorpholism exists. Homology to genome of homology arms is important in the frequency of gene targeting, and a difference in base sequence sometimes leads to a significant lowering in gene targeting frequency (NPL 7: Datt, A. et al., (1997) Proc. Natl. Acad. Sci. U.S.A. 94, 9757-9762, NPL 8:Selva E. M. et al., (1995) Genetics. 139, 1175-1188, NPL 9: Riele H. et al., (1992) Proc. Natl. Acad. Sci. U.S.A. 89, 5128-5132, NPL 10:Deng C, D, Capecchi M, R (1992) Mol. Cell. Biol. 12, 3365-3371). Accordingly, in female-derived culture cells such as CHO cells, the influence of polymorpholism of X chromosomes can also render the frequency of acquisition of recombinant cells lower than that in male-derived culture cells.
Thus, the development of a technique for efficiently generating recombinant CHO cells that express a target protein gene is still demanded.

### [Citation List]

### [Patent Literature]

[PTL1] JP 2002-541854A
[PTL 2] JP 9(1997)-510865A
[PTL 3] JP 2001-516221A
[PTL 4] WO 2004/022741A
[PTL 5] JP 2007-325571A
[PTL 6] JP 5(1993)-507853A

### [Non Patent Literature]

[NPL1] Kim, N. S. (1998) Biotechnol. Bioeng., 60, 679-688.
[NPL 2] Brown, M. E. et al. (1992) Cytotechnology, 9, 231-236.
[NPL.3] Barnes, L. M. et al. (2003) Biotechnology and Bioengineering, 81, 631-639.
[NPL.4] Koyama Y Et Al., (2006) Biotechnology And Bioengineering, 95, 1052-1060
[NPL 5] Porter C. G. Itzhaki J. E, Eur. J. Biochem 218, 273-281
[NPL.6] Annual Report of the Hiroshima University Research Institute for Radiation Biology and Medicine, vol. 44 (2003)
[NPL 7] Datt, A. et al., (1997) PNAS, U.S.A. 94, 9757-9762
[NPL 8] Selva E. M. et al., (1995) Genetics. 139, 1175-1188
[NPL 9] Riele H. et al., (1992) Proc. Natl. Acad. Sci. U.S.A. 89, 5128-5132
[NPL 10] Deng C, D, Capecchi M, R (1992) Mol. Cell. Biol. 12, 3365-3371
[NPL 11] PNAS,88, 9488-9502(1991)
[NPL 12] Somatic Cell. Mol. Genet., 19, 363-375
[NPL 13] PNAS,86,4574-4578(1989)
[NPL 14] Mol Gen. Genet., 212, 301-309(1988)
[NPL 15] Molecular Biology Reports, 31, 85-90(2004)
[NPL 16] Biotechnology and Bioengineering, 91, 1-11(2005)

### [Summary of Invention]

The present inventors have found that recombinant CHO cells can be generated with significantly high frequency by determining a complete DNA sequence including an intron in an hprt gene of CHO cells and further introducing a target protein gene into CHO cells by a specific DNA construct containing a homologous DNA fragment of the hprt gene. The present invention has been made based on such finding.
Accordingly, an object of the present invention is to provide a DNA construct useful for efficient generation of recombinant CHO cells and a process for generating recombinant CHO cells using the same.

According to one aspect of the present invention, there is provided a DNA construct comprising, from a 5' end toward a 3' end, a first homologous DNA fragment, a target protein gene, and a second homologous DNA fragment,
the first and second homologous DNA fragments having homology allowing for homologous recombination with a part of a hypoxanthine-phosphoribosyltransferase enzyme (hprt) locus in a CHO cell genome and having a chain length of not less than 1 kbp.

According to another aspect of the present invention, there is provided a process for generating recombinant CHO cells, the process comprising introducing the vector containing the DNA construct into CHO cells.

According to the present invention, recombinant CHO cells that express target protein genes can be acquired with significantly high frequency.

### [Brief Description of Drawings]

[Fig. 1] Fig. 1 is a typical diagram of an hprt locus of a CHO-K1 cell line in Example 1. Nos. 1 to 9 represent exons 1 to 9 of the hprt gene, respectively, and sequences between the exons represent introns 1 to 8.
[Fig. 2] Fig. 2 is a typical diagram of a vector for homologous recombination used in Example 2. Fig. 2 (A) shows a vector containing a homologous DNA fragment derived from an hprt gene of CHO cells. Fig. 2 (B) shows a vector containing a homologous DNA fragment derived from human HT1080 cells.
[Fig. 3] Fig. 3 (A) is a typical diagram showing a relationship among an hprt gene region of CHO cells, a vector for homologous recombination, and DNA as an index of genome PCR of recombinant CHO cells in Example 4. Figs. 3 (B) and 3 (C) are diagrams showing the results of PCR in Example 4.
[Fig. 4] Fig. 4 (A) is a typical diagram showing a relationship among an hprt gene region of CHO cells, a vector for homologous recombination, and DNA as an index of Southern hybridization in Example 5. Figs. 4 (B) shows the results of Southern hybridization in Example 5.
[Fig. 5] Fig. 5 is a graph showing the amount of antibody produced by recombinant CHO cells acquired in Example 6.
[Fig. 6] Fig. 6 is a typical diagram of a vector for homologous recombination having a 2.5-kbp homologous DNA fragment used in Example 7.
[Fig. 7] Fig. 7 (A) is a typical diagram showing a relationship among a hprt gene region of CHO cells, a vector for homologous recombination, and DNA as an index of genome PCR of recombinant CHO cells in Example 7. Figs. 7 (B) and (C) show the results of PCR in Example 7.
[Fig. 8] Fig. 8 (A) is a typical diagram showing a relationship among a hprt gene region of CHO cells, a vector for homologous recombination, and DNA as an index of Southern hybridization in Example 7. Fig. 8 (B) is a diagram showing the results of Southern hybridization in Example 7.
[Fig. 9] Fig. 9 (A) is a typical diagram for explaining details of genome PCR for the confirmation of remaining of a wild-type hprt gene in CHO cells. The drawing located left in Fig. 9 (A) shows PCR in wild-type CHO cells, the drawing located at the center of Fig. 9 (A) shows PCR in heterojunction-type recombinant CHO cells, and the drawing located right in Fig. 9 (A) shows PCR in homojunction-type recombinant CHO cells. Fig. 9 (B) shows the results of PCR in Example 8.
[Fig. 10] Fig. 10 is a typical diagram of a vector for homologous recombination that has a 2.5-kbp homologous DNA fragment and a CHO endogenous promoter used in Example 9.
[Fig. 11] Fig. 11 is a diagram showing the productivity of antibody in Example 9 where acquired recombinant CHO cells are subcultured for a long period of time.
[Fig. 12] Fig. 12 is a typical diagram showing a vector for homologous recombination used in Example 10 that has a 2.5-kbp homologous DNA fragment.

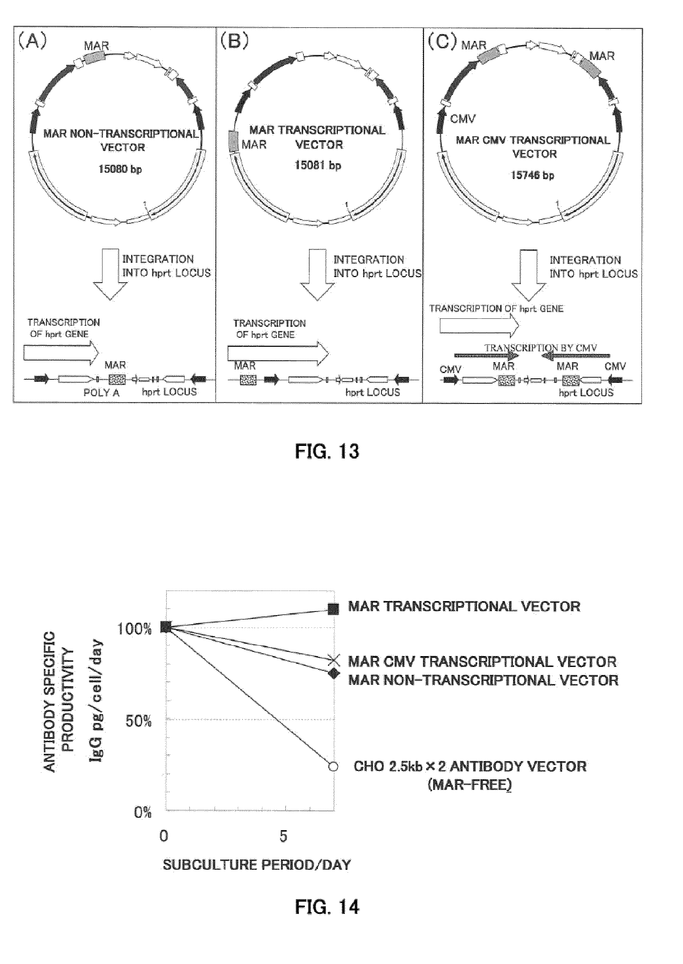

[Fig. 13] Fig. 13 is a typical diagram showing a CHO recombinant vector having MAR derived from a human hprt gene intron and a chromosome comprising the vector integrated into a CHO hprt locus used in Example 11. Fig. 13 (A) shows a vector having an MAR non-transcriptional structure and a chromosome structure after the integration. Fig. 13 (B) shows a vector having a structure that MAR is transcribed by transcription of an hprt locus and a chromosome structure after the integration. Fig. 13 (C) shows a vector having a structure that MAR is transcribed by a CMV promoter and a chromosome structure after the integration.
[Fig. 14] Fig. 14 is a graph showing stability in antibody production of CHO cells acquired by a CHO recombinant vector having MAR used in Example 11.
[Fig. 15] Fig. 15 is a typical diagram showing a CHO recombinant vector having a homology arm length of 200b×2 (A) and a CHO recombinant vector having a homology arm length of 500b×2 (B) used in Example 12.
[Fig. 16] Fig. 16 is a typical diagram showing a CHO recombinant vector having a homology arm length of 5kb×2 used in Example 13.

### [Description of Embodyments]

### DNA construct

One feature of the DNA construct according to the present invention is to comprise two homologous DNA fragments that have homology allowing for homologous recombination with a part of hprt locus in a CHO cell genome and each have a chain length of not less than 1 kbp. It is surprising that, according to the DNA construct, recombinant CHO cells can be acquired with significantly high frequency despite the fact that CHO cells have two X chromosomes. According to the DNA construct of the present invention, as demonstrated in Example 10 which will be described later, 130 recombinant cells are obtained per 1x10⁻⁷ cells. As described above, since the estimated probability of simultaneous recombination in hprt locuses of the two chromosomes is theoretically 10⁻¹², this result is a surprising fact.

In the DNA construct according to the present invention, a part of the hprt locus is a target region of homologous recombination. The adoption of a part of the hprt locus as the target region is advantageous in stably expressing the target protein gene on a high level. Further, the integration of the DNA construct into the target region is also preferred from the viewpoints of inhibiting the transcription and expression of the hprt gene to inactivate the function of the hprt gene and efficiently acquiring recombinant cells by negative selection using 6-TG or the like.

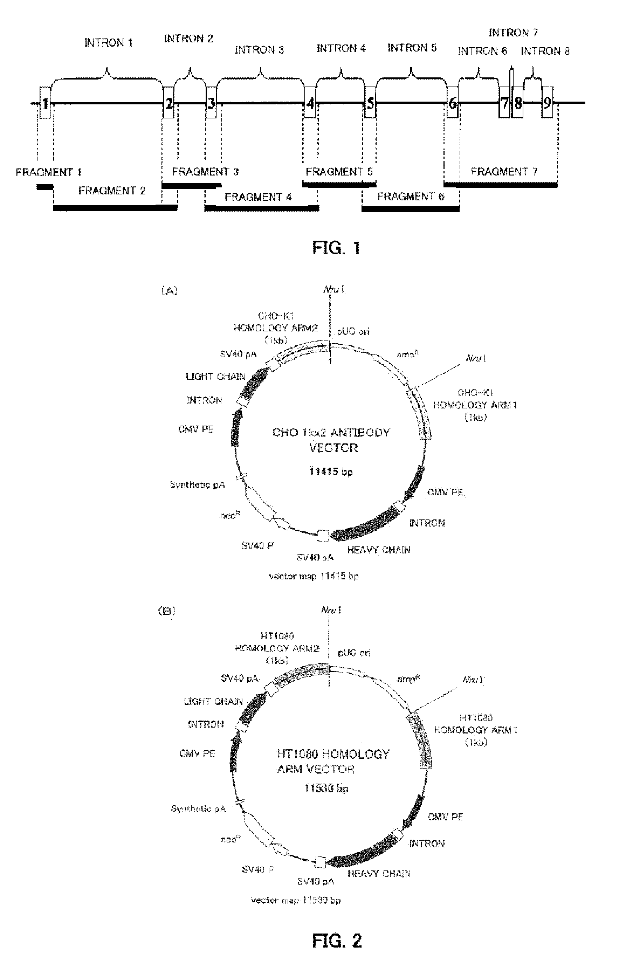

The target region in the present invention may be properly determined in the hprt locus as long as the expression of the target protein gene is not inhibited. When the chain length and the like of homologous DNA fragments necessary for homologous recombination are taken into consideration, the target region is preferably a region containing at least a part of introns of the hprt gene. For introns, the present inventors have now determined base sequences. Specifically, introns 1 to 8 shown in Fig. 1 which will be described later, may be mentioned that have a base sequence represented by any of SEQ ID Nos. 15 to 22.

The target region according to the present invention may contain the whole or a part of exons adjacent to the introns. Specifically, exons 1 to 9 shown in Fig. 1 may be mentioned as the exon. Base sequences thereof can be acquired by access to known database, for example, in National Center for Biotechnology Information of USA.

In the present invention, homologous DNA fragments homologous to a desired region in the hprt locus may be properly constructed by a person having ordinary skill in the art based on information about the arrangements of and their base sequences of introns and exons in the hprt locus shown in Fig. 1.
The DNA construct according to the present invention having the homologous DNA fragments can be integrated into a desired target region in the hprt locus. Embodiments of the integration include (1) a type in which exons are divided by the integration of the DNA construct, (2) a type in which one or more exons are deleted by the integration of the DNA construct, (3) a type in which one exon is amplified to two by the integration of the DNA construct and the DNA construct is inserted into between the two exons, (4) a type in which introns are divided by the integration of the DNA construct, (5) a type in which one or more introns are deleted by the integration of the DNA construct, (6) a type in which one intron is increased to two by the integration of the DNA construct and the DNA construct is inserted into between the two introns.

When the chain length necessary for homologous recombination is taken into consideration, as described above, the DNA fragment according to the present invention is preferably homologous to a region containing at least a part of introns of the hprt gene. Accordingly, in one embodiment of the present invention, the first homologous DNA fragment and the second homologous DNA fragment according to the present invention comprise a base sequence described in any of SEQ ID Nos. 15 to 22 or a partial sequence thereof.

The lower limit of the chain length of the partial sequence is 1 bp, and the upper limit can be properly regulated in a range of chain lenghs of base sequences described in any of SEQ ID Nos. 15 to 22.

The homology between the homologous DNA fragments and the hprt locus may be properly determined by taking into consideration of the efficiency of homologous recombination but is preferably not less than 99.0%, more preferably not less than 99.9%, still more preferably 100%. The homology may be properly determined, for example, by analysis with DNA sequencer or the like.

Further, the homologous DNA fragments have a chain length of not less than 1 kbp, and this chain length is preferred in achieving homologous recombination of the DNA construct with significantly high frequency. The lower limit of the chain length of the homologous DNA fragments is preferably not less than 2.5 kbp. The upper limit of the chain length of the homologous DNA fragments is not more than 7.5 kbp, preferably not more than 5 kbp. The upper limit and the lower limit of the chain length of the homologous DNA fragments may be properly combined to define the chain length range of the homologous DNA fragments. Specifically, the chain length is preferably 1 kbp to 7.5 kbp, more preferably 1 kbp to 5kbp, most preferably 2.5 kbp to 5 kbp. When the chain length is in the above-defined range, the frequency of acquisition of the recombinant cells can be kept good while achieving homologous recombination of the DNA construct with significantly high frequency.

In the DNA construct according to the present invention, the target protein gene preferably codes for a protein useful as a medicine. cDNA-derived sequences or structural genes containing natural introns derived from genome DNA may be suitably utilized as a target protein gene. Specific examples of target proteins include antibodies, enzymes, cytokines, hormones, coagulation factors, regulatory proteins, and receptors. Preferred are monoclonal antibodies, polyclonal antibodies, erithropoietins, and tissue-specific plasminogen activators or granulocyte colony activators.

Preferably, the target protein gene is integrated as an expression unit containing elements necessary for expression of such as promoter sequences and transcription termination signal sequences into the hprt locus. In one embodiment of the present invention, the target protein gene is disposed as an expression unit containing at least a promoter sequence and a transcription termination signal sequence in the DNA construct.
A construction may also be adopted in which the element necessary for expression may be endogenous in CHO cells. This embodiment falls within the scope of the present invention.

The promoter or the transcription termination signal may be properly determined depending, for example, upon the type and properties of the target protein gene. Examples of suitable promoter sequences include CMV promoters and SV40 promoters. Examples of suitable transcription termination signal sequences include BGH poly A signal sequences and SV40 poly A signal sequences.

For example, regulatory elements for efficient expression of a target gene (for example, enhancers, IRES (internal ribosome entry site) sequences, LoxP sequences, FRT sequences or other recombinant enzyme recognition sequences) may be properly selected and used as the element necessary for expression other than the promoter sequence and the transcription termination signal sequence. The regulatory element may be disposed at a proper position in the expression unit depending upon the properties. The element necessary for expression may be properly selected by taking into consideration, for example, the productivity of the target protein.

Preferably, the DNA construct comprises, in addition to the above elements, a positive selective marker gene. The positive selective marker gene may be properly disposed in the DNA construct as long as the homologous recombination is not inhibited. Examples of suitable positive selective marker genes include neomycin-resistant genes, hygromycin resistant genes, Zeocin resistant genes, dihydrofolate reductase genes, and glutamine synthase genes. The use of the positive selective marker gene is advantageous in that, in the selection of recombinant CHO cells, both positive selection and negative selection by inactivation of the hprt gene can be applied and, thus, false-positive clones can be significantly reduced.

### Vector

The DNA construct according to the present invention can be integrated into the vector, followed by introduction into a CHO cell genome. The vector system is not particularly limited as long as the DNA construct can be integrated into the CHO cell genome by a homologous recombination reaction. Preferred are plasmid vectors, cosmid vectors, phage vectors, or artificial chromosome vectors.

The DNA construct according to the present invention and the vector comprising the DNA construct are suitably constructed by a combination of a restriction enzyme cleaving reaction and a ligation reaction. A DNA construct and a vector comprising the DNA construct can be constructed, for example, by incorporating a restriction enzyme recognition sequence at both ends of each constituent unit, performing a cleaving reaction with a restriction enzyme for the recognition sequence, removing unnecessary DNA sequences (for example, operating sequences within E. coli) by taking-off of gel, and performing a ligation reaction of the resultant unit.

The vector DNA constructed by the ligation reaction is purified, for example, by phenol-chloroform extraction and can be grown in host cells such as E. coli. or yeasts selected while taking into consideration of, for example, the type of vectors.

Preferably, the vector according to the present invention mounts a CHF1α promoter operatively ligated to the target protein gene. The expression of a foreign genes integrated into the CHO hprt locus occurs through the inactivation of the promoter. When a vector has the structure that causes expression of the target protein gene by the CHEF1α promoter endogenous in CHO, stable expression in the CHO hprt locus is possible.

Preferably, in the vector according to the present invention, after the integration of the vector into a chromosome, a nucleus/matrix adhesion region (MAR) derived from the first intron of the human hprt gene is maintained in such structure that MAR per se is transcribed by transcription that originally occurs at the integration site. When MAR of the human hprt gene intron is mounted on the vector in the above structure, recombinant CHO having expression stability can be efficiently generated.

The present applicants have previously reported that a foreign gene integrated into an hprt locus is stably expressed in an HT1080 cell line, a human-derived cell line (PTL 4: WO 2004/022741 and NPL 5: Porter C. G. Itzhaki J. E, Eur. J. Biochem 218, 273-281). Further, it is known that a stabilizing factor called a nucleus/matrix adhesion region (MAR) is present in the first intron of the human hprt gene (NPL 14). From these facts, it is estimated that the stabilization of expression in the human hprt gene is realized by the contribution of MAR present around the integration site.

By the way, the hprt gene is a gene that is common in many mammals including CHO. In the hprt locus of CHO, it is estimated that difficulties are encountered in selecting integration cells. Accordingly, examples of cases where the hprt locus has been used as vector integration sites are not known. It is common for a person having ordinary skill in the art to consider that, when a foreign gene is integrated into the hprt locus of CHO, stable expression is possible as in human cells. However, the intron sequence of the hprt locus of CHO is utterly different from that of the hprt gene of human, and a surprising fact that MAR is absent in introns has been elucidated for the first time by the present inventors. In fact, as demonstrated in Example 11, the foreign gene integrated into the hprt locus of CHO could not be stably expressed.

From the above results, when a foreign gene is integrated into the hprt locus of CHO, a person having ordinary skill in the art would consider that, in CHO, stable expression is possible as in human cells by acquiring MAR of the human hprt gene intron, mounting MAR on a CHO recombinant vector, and integrating the vector into the hprt locus of CHO. In line with this way of thinking, related art are known that partial stabilization has been realized by mounting MAR on a vector and randomly integrating the vector into a chromosome of CHO or holding the vector as an extrachromosomal plasmid (NPL 14: Mol Gen. Genet., 212, 301-309 (1988) and NPL 15: Molecular Biology Reports, 31, 85-90 (2004)). In these related art , a structure in which MAR is mounted on the vector at its position where MAR per se is not transcribed (NPL 14) or a structure in which MAR per se is also transcribed by a promoter disposed with an expectation of the target protein gene transcription (NPL 15) is adopted.

However, as demonstrated in Example 11 which will be described later, unexpectedly, in the hprt locus of CHO, these structures offer no significant effect and thus are unsatisfactory for long-term stable expression. It has been found that, in order to achieve stable expression in the hprt locus of CHO, as demonstrated in Example 11, a necessary structure is that MAR mounted on the vector is transcribed only by transcription that originally occurs at the vector integrated site.

### Recombinant CHO cells/generation process

The recombinant CHO cells according to the present invention can be suitably generated by introducing the vector into CHO cells.
Accordingly, the recombinant CHO cells according to the present invention comprise an exogenous target protein gene integrated into hprt locus. In a preferred embodiment of the present invention, in the recombinant CHO cells, the function of the hprt gene is inactivated. The recombinant CHO cells are advantageous in stably producing target proteins such as antibodies on a high level.

### Introduction of vector

Commonly used methods are suitable for the introduction of the vector. Examples of such methods include a calcium phosphate method, an electroporation method, a microinjection method, DEAE-dextran method, a method using a liposome reagent, and a lipofection method using a cationic lipid. When the vector is cyclic, a method may also be adopted in which the vector is linearized by a conventional method and the linearized vector is then introduced into cells.

Depending upon properties of the vector or the target protein, in consideration of acquisition efficiency and the like of recombinant cells, a site-specific introduction system utilizing a recombinant enzyme such as a Cre/LoxP system or an Flp/FRT system may also be prooperly applied. This embodiment also falls within the scope of the present invention.

### Screening of cell line

In the generation process according to the present invention, after the introduction, screening of recombinant CHO cells is preferably carried out. The step of screening can be carried out by negative selection based on inactivation of the hprt gene. When a positive marker gene has been introduced into recombinant CHO cells, screening of cells can be carried out with high accuracy by a combination of the negative selection with the positive selection.

In addition to the above screening methods, for example, a promoter trap method and a poly A trap method may be properly used in combination.

Further, in the generation method according to the present invention, culture under serum-free conditions is possible by acquiring recombinant CHO cells and then naturalizing the recombinant CHO cells to a chemical defined medium or the like. Conditions for the naturalization may be properly determined depending upon the state of recombinant cells.

### Process for producing target protein

According to another aspect of the present invention, there is provided a process for producing a target protein, the process comprising providing the recombinant CHO cells and culturing the cells to produce a target protein. According to this process, the target protein can be acquired in an efficient and stable manner.

The medium used in the step of culture may be properly selected from conventional media depending upon the state of recombinant CHO cells but is preferably serum-free medium. When the culture cost and the purification cost are taken into consideration, preferably, the medium is not supplemented with a selective drug. Examples of suitable media include chemical defined media.

Conventional culture methods such as batch culture method, fed batch culture method, and reflux culture method are applicable as the culture method.

Various methods used in the generation of recombinant CHO cells are described in more detail, for example, in F.M. Ausubel, Current Protocols in Molecular Biology, John Wiley & Sons, New York, (1989), the disclosure of which is incorporated by reference herein.

### [Examples]

The present invention is further illustrated by the following Examples that are not intended as a limitation of the invention.
In the following experiments, conditions for reactions such as reactions by restriction enzymes, PCR reactions, and ligation reactions were set by reaction conditions recommended by manufacturers or methods described in Molecular Cloning 2nd Edition; Sambrook et al., Cold Spring Harbor Laboratory Press. For various plasmid vector DNAs obtained, DNA sequences were determined with a DNA sequencer (310 Genetic Analyser Applied Bio Systems, Inc.). Homology arms 1 and 2 correspond to first and second homologous DNA fragments according to the present invention, respectively.

### Example 1: Acquisition of information on hprt locus DNA sequence of CHO cells

A CHO-K1 cell line, a Chinese hamster oocyte cell line, obtained from JCRB Cell Bank (Cell No; JCRB9018) was cultured in a CO₂ incubator (37°C, 5% CO₂) using an AMEM medium (composition; Advanced MEM (GIBCO), 5% [v/v] FBS, 1× GlutaMAX (GIBCO)). The culture solution thus obtained was centrifuged to obtain CHO-K1 cell pellets. The pellets were treated with DNA Isolation Kit for Cells and Tissues (Roche Diagnostics K.K.) to obtain genome DNA. Seven DNA fragments of hprt locus of CHO-K1 cells were obtained by PCR (KOD-Plus ver.2, TOYOBO) using the genome DNA as a template. The fragments were as shown in Fig. 1. PCR primers that had been used for the amplification of the 7 fragments were prepared by reference to primer sequences described in Zu Z et al. Mutat Res. 1993. 288(2): 237-48. The primer sequences were as follows.

Fragment 1 sense primer:
   5'- tctgcaggct tcctcctcac accg -3' (SEQ ID No. 1)
Fragment 1 antisense primer:
   5'- acatgtcaag gcaacgccat ttcca -3' (SEQ ID No. 2)
Fragment 2 sense primer:
   5'- tggaaatggc gttgccttga catgt -3' (SEQ ID No. 3)
Fragment 2 antisense primer:
   5'- caccttttcc aaatcctcga -3' (SEQ ID No. 4)
Fragment 3 sense primer:
   5'- agcttatgct ctgatttgaa atcagctg -3' (SEQ ID No. 5)
Fragment 3 antisense primer:
   5'- cttcagtctg ataaaatcta cagtca -3' (SEQ ID No. 6)
Fragment 4 sense primer:
   5'- aagacttgcc cgagatgtca tgaa -3' (SEQ ID No. 7)
Fragment 4 antisense primer:
   5'- ccaagtgagt gattgaaagc acag -3' (SEQ ID No. 8)
Fragment 5 sense primer:
   5'- tgtgtgtatt caagaatatg catg -3' (SEQ ID No. 9)
Fragment 5 antisense primer:
   5'- gctgagaaaa tttaacagta ttttag -3' (SEQ ID No. 10)
Fragment 6 sense primer:
   5'- caaatacaag caagaatttc ccagag -3' (SEQ ID No. 11)
Fragment 6 antisense primer:
   5'- ggacttgaac atctagggag -3' (SEQ ID No. 12)
Fragment 7 sense primer:
   5'- acttaccact taccattaaa tacc -3' (SEQ ID No. 13)
Fragment 7 antisense primer:
   5'- gacaatctat cgaaggctca tagtgc -3' (SEQ ID No. 14)

DNA sequencing was carried out for the 7 fragments (BigDye Terminator v3.1, Applied Biosystems, Inc.).
As shown in Fig. 1, DNA sequences of
intron 1 located between exon 1 and exon 2 (SEQ ID No. 15),
intron 2 located between exon 2 and exon 3 (SEQ ID No. 16),
intron 3 located between exon 3 and exon 4 (SEQ ID No. 17),
intron 4 located between exon 4 and exon 5 (SEQ ID No. 18),
intron 5 located between exon 5 and exon 6 (SEO ID No. 19),
intron 6 located between exon 6 and exon 7 (SEQ ID No. 20),
intron 7 located between exon 7 and exon 8 (SEQ ID No. 21), and
intron 8 located between exon 8 and exon 9 (SEQ ID No. 22) of hprt locus in a CHO-K1 cell line were determined.

### Example 2: Construction of targeting vector

In order to integrate an antibody gene into the hprt locus, a vector (a CHO 1 kx2 antibody vector) including an antibody gene and represented by Fig. 2(A) was constructed by the following method.
Further, for comparison, a vector (HT1080 homology arm vector) that has the same construction as the CHO 1kx2 antibody vector except for the substitution of a homologous DNA fragment derived from an hprt gene sequence in CHO cells for a homologous DNA fragment derived from a human hprt gene sequence and is represented by Fig. 2(B) was constructed.

### Acquisition of DNA fragment homologous to human hprt gene

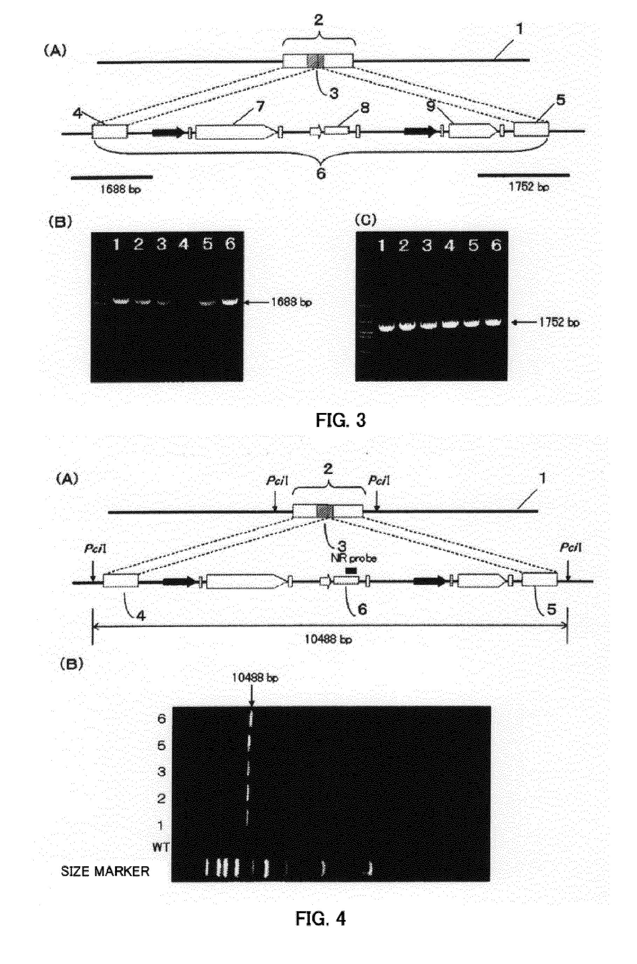

An HT1080 cell line, a human-derived cell line, obtained from JCRB Cell Bank (catalog number: IF050354) was treated with a GFX Genomic Blood DNA Purification Kit (Amersham Biosciences) to obtain genome DNA. Next, DNA fragments (HA1 and HA2) homologous to an hprt gene as a target were cloned by a PCR reaction (KOD-Plus-, TOYOBO) using the genome DNA as a template. As shown in Fig. 3 which will be described later, HA1 and HA2 were set to sequences homologous to a region including exon 3 in the hprt gene. The following primer sequences were used in the PCR reaction.

HA1 sense primer. 5'-CCTGCAGGTCGCGATTGGTACTTGTTCAGCTTTATTCAAG-3' (SEQ ID No. 23)
HA1 antisense primer: 5'-GTCGACAAGGACGCGTTCTGATAAAATCTACAGTCATAGGA-3' (SEQ ID No. 24)
HA2 sense primer: 
5'-GTCGACCTCTAGCTAGAGCTTGGCGTAATCATGGTCTCCGGAGACTGAAGA
GCTATTGTGTGAGTAT-3' (SEQ ID No. 25)
HA2 antisense primer: 5'-ACATGTTCTCTTAAGTCGCGAAGTAGTGTTATGATGTATGGGCATA-3' (SEQ ID No. 26)

In the PCR reaction, a recognition site of restriction enzymes Sse 83871 and Nru I was added to the 5' end of the HA1 sense primer. Likewise, a recognition site of Sal I and Mlu I was added to the 5' end of the HA1 antisense primer, a recognition site of Sal I and Acc III was added to the 5' end of the HA2 sense primer, and a recognition site of Pci I and Nru I was added to the 5' end of the HA2 antisense primer.

A DNA sequence containing an ori sequence, which is a replication origin within E. coli (Escherichia coli), and an ampicillin-resistant gene was cloned by a PCR reaction from DNA of a pQBl25 plasmid vector (Wako Pure Chemical Industries, Ltd.). The following primer sequences were used in the PCR reaction. In the PCR reaction, a recognition site of restriction enzymes Pci I and Sse 83871 was added to the 5' end and the 3' end of the sense primer.

Ecoli sense primer: 5'-ACATGTGAGCAAAAGGCCAGCAAAAGGCCAGGAAC-3' (SEQ ID No. 27)
Ecoli antisense primer: 5'-CCTGCAGGGACGTCAGGTGGCACTTTTCGGGGAAATGTGC-3' (SEQ ID No. 28)

HA1, HA2, and a DNA sequence containing an ori sequence and an ampicillin-resistant gene were cloned by PCR reaction, respectively. These three DNA sequences were cleaved with restriction enzymes Pci I, Sse 83871, and Sal I, and subjected to a ligation reaction to obtain a pHA12 plasmid vector. The pHA12 plasmid vector was then cleaved with restriction enzymes Mlu I and Acc III to obtain DNA sequence 1 in which HA2, the DNA sequence containing the ori sequence and the ampicillin resistance gene, and HA1 were linked in that order from the 5'-end.

Further, a DNA fragment containing a restriction enzyme Bam HI recognition site was obtained from pcDNA3,1-Hygro(+) (Invitrogen). Restriction enzymes Mlu I and Acc III sites were added to the end, followed by PCR amplification to obtain DNA sequence 2. This fragment was linked to DNA sequence 1 to obtain a p23HA12 plasmid vector.

### Construction of basic vector

The p23HA12 plasmid vector was cleaved with Mlu I and Bam] HI. A DNA fragment obtained by subjecting linker 2-s oligo and linker 2-a oligo to annealing was inserted thereinto to obtain a basic vevtor II.

Linker 2-s oligo: 5'-CGCGTatcTCTAGAataATCGATagaAAGCTTacaG-3' (SEQ ID No. 29) Linker 2-a oligo
5'- GATCCtgtAAGCTTtctATCGATtatTCTAGAgatA-3' (SEQ ID No. 30)

A DNA sequence 3 containing an SV40 promoter, a poly A signal sequence, and a neomycin-resistant gene (an expression cassette containing SV40, neo R, and synthetic poly A) was amplified by a PCR reaction from DNA of a pQBl15 plasmid vector (Wako Pure Chemical Industries, Ltd.). The following primer sequences were used in the PCR reaction. In the PCR reaction, a recognition site of a restriction enzyme Xba I was added to the 5' end of the sense primer, and a recognition site of synthetic poly A and Cla I was added to the 3' end of the sense primer.

neo R sense primer: 5'- ccttTCTAGActtctgaggcggaaagaacc-3' (SEQ ID No. 31)
neo R antisense primer:

5’-cttATCGATtCACACAAAAAACCAACACACAGATGTAATGAAAATAAAGATATTTT
ATTgtgggcgaagaactccagca-3’ (SEQ ID No. 32)

The basic vector II was cleaved with restriction enzymes Xba I and Cla I, and DNA sequence 3 was ligated thereto to obtain a basic vector+Neo R plasmid.

### Construction of antibody heavy chain expression cassette

A variable region was amplified by PCR with a primer set of IGH-3A21s and IGH-3A21a1 using a plasmid holding a heavy chain variable region ("Cloning of cDNA and Characterization of Anti-RNase A Monoclonal Antibody 3A21": Journal of Fermentation and Bioengineering. Vol. 82, No. 3, pp. 312-314,1999) as a template to obtain DNA sequence 4.

IGH-3A21 s primer: 5'- TAAAAGGTGTCCAGGATGTGCAGTTTCAGG -3' (SEQ ID No. 33)
IGH-3A21a1 primer: 5'- GAGGCCGATGAAACAGTGACCAGAGTCCCT -3' (SEQ ID No. 34)

RNA extracted from RPMI8226 culture cells with ISOGEN (NIPPON GENE CO., LTD.) was used as a template, and RT-PCR was performed with One-step RT-PCR kit (QIAGEN). A constant region was amplified by PCR with a primer set of IGH-Cs and IGH-Ca using the resultant mRNA as a template to obtain DNA sequence 5.

IGH-Cs primer: 5'- CATCGGCCTCCACCAAGGGCCCATCGGTCT -3' (SEQ ID No. 35)
IGH-Ca primer: 5'- TTAAGCGGCCGCTCATTTACCCGGAGACAG -3' (SEQ ID No. 36)

PCR amplification was then performed with a primer set of IGH-LS and IGH-Va using DNA sequence 4 as a template to obtain DNA sequence 6 with a secretion signal added thereto.

IGH-LS primer: 
5’-GGTCGCCACCATGGAGTTTGGACTGAGCTGGGTTTTCCTTGTTGCTATTTTA
AAAGGTGTCCAGGATGTG-3’ (SEQ ID No. 37)
IGH-Va primer: 5'-GCCCTTGGTGGAGGCCGATGAAACAGTGAC -3' (SEQ ID No. 38)

Assemble PCR was performed with a primer set of IGH-Vs and IGH-Ca using DNA sequence 5 and DNA sequence 6 as a template to obtain DNA sequence 7 comprising both the sequences linked to each other.

IGH-Vs primer: 5'-TTCCGGTACCGGTCGCCACCATGGAGTTTG -3' (SEQ ID No. 39)

A pmaxGFP plasmid (amaxa) was subjected to a PCR reaction for the introduction of mutation with a Notl-site-s/Notl-site-a primer set using Prime STAR MAX (Takara Shuzo Co., Ltd.) to obtain a pmaxGFP+Notl plasmid containing a Notl site introduced just before the CMV promoter.

Notl-site-s primer: 5'-ATGCggccgcATGTCAATATTGGCCATT-3' (SEQ ID No. 40)
Notl-site-a primer: 5'-GACATgcggccGCATGGGAGGAGACCGGG -3' (SEQ ID No. 41)

A 0.5-kbp fragment was then amplified with a Notl-spacerA-s/Notl-spacerA-a primer set using pcDNA3.1-hygro(+) (Invitrogen) as a template and was inserted into the Norl site of pmaxGFP+Notl to obtain a pmaxGFP+spacerA plasmid.

Notl-spacerA-s primer: 5'-ttGCGGCCGCgaaaaagcctgaactcaccg -3' (SEQ ID No. 42)
Notl-spacerA-a primer: 5'-ttGCGGCCGCgacggtgtcgtccatcacag -3' (SEQ ID No. 43)

A heavy chain was amplified with a Kpnl-IGH-s/BgIII-IGH-a primer set using DNA sequence 7 as a template, followed by replacement with GFP in pmaxGFP+spacerA to obtain a pmax+spacerA+IgH plasmid.

Kpnl-IGH-s primer: 
5'-ccttGGTACCGAAGCCGCTAGCGCTACCGGTCGCCACCaTGGAGTTTGGACT
GAGCTGGG-3' (SEQ ID No. 44)
BgIII-IGH-a primer: 5'-ccttAGATCTtcatttacccggagacaggg-3' (SEQ ID No. 45)

PCR amplification was performed with a MluI-spA(IgH)-s2/MluI-CMV-IgHL-SVpA-a primer set using pmax+spacerA+IgH as a template. The amplification product was cloned into a pCR-Bluntll-TOPO vector (Invitrogen), followed by cleaving with a restriction enzyme Mlu I to obtain DNA sequence 8 (heavy chain cassette).

MluI-spA(IgH)-s2 primer: 5'-ccttACGCGTgacggtgtcgtccatcacag -3' (SEQ ID No. 46)
MluI-CMV-IgHL-SVpA-a primer: 5'-cttACGCGTAACGTCTCGCCCTTTGGTCTC -3' (SEQ ID No. 47)

### Construction of antibody light chain expression cassette

Likewise, a plasmid holding a light chain variable region was provided as a template, and the variable region was amplified by PCR with a primer set of IGL-3A21s1 and IGL-3A21a1 to obtain DNA sequence 9.

IGL-3A21s1 primer: 5'-CCCAGGTGCCAGATGTGACATCAAGATGAC-3' (SEQ ID No. 48)
IGL-3A21a1 primer: 5'-GCCACAGTTCGTTTTATTTCCAACTTTGTC-3' (SEQ ID No. 49)

RNA extracted from RPMI8226 culture cells with ISOGEN was provided as a template, and RT-PCR was performed with One-step RT-PCR kit. A constant region was amplified by PCR with a primer set of IGL-Cs and IGL-Ca using the resultant mRNA as a template to obtain DNA sequence 10.

IGL-Cs primer: 5'-TGGAAATAAAACGAACTGTGGCTGCACCAT-3' (SEQ ID No. 50)
IGL-Ca primer: 5'-TTAAGCGGCCGCCTAACACTCTCCCCTGT-3' (SEQ ID No. 51)

PCR amplification was performed with a primer set of IGL-LS and IGL-3A21a using DNA sequence 9 as a template to obtain DNA sequence 11 with a secretion signal added thereto.

IGL-LS primer: 
5’-GGTCGCCACCATGGACATGAGGGTCCCCGCTCAGCTCCTGGGGCTCCTGC
TGCTCTGGCTCCCAGGTGCCAGATGTGACA -3’ (SEQ ID No. 52)
IGL-3A21a primer: 5'-GCCACAGTTCGTTTTATTTCCAACTTTGTC-3' (SEQ ID No. 53)

Assemble PCR was performed with a primer set of IGL-Vs and IGL-Ca using DNA sequence 10 and DNA sequence 11 as a template to obtain DNA sequence 12 comprising both products linked to each other.

IGL-Vs primer: 5'-CCTTGGTACCGGTCGCCACCATGGACATGA -3' (SEQ ID No. 54)

A 0.5-kbp fragment was then amplified with a Notl-spacerB-s/Notl-spacerB-a primer set using pcDNA3.1-hygro(+) (Invitrogen) as a template and was inserted into the Norl site of pmaxGFP+Notl to obtain a pmaxGFP+spacerB plasmid.

Notl-spacerB-s primer: 5'-ttGCGGCCGCctgtgatggacgacaccgtc-3' (SEQ ID No. 55)
Notl-spacerB-a primer: 5'-ttGCGGCCGCctattcctttgccctcggac-3' (SEQ ID No. 56)

A light chain was then amplified with a Kpnl-IGH-s/Bglll-IGH-a primer set using DNA sequence 12 as a template, followed by replacement with GFP in pmaxGFP+spacerB to obtain a pmax+spacerB+IgL plasmid.

Kpnl-IGL-s primer: 
5'-ccttGGTACCGAAGCCGCTAGCGCTACCGGTCGCCACCatggacatgagggtcccc
gc-3' (SEQ ID No. 57)
BgIII-IGL-a primer: 5'-ccttAGATCTctaacactctcccctgttga -3' (SEQ ID No. 58)

PCR amplification was performed with a HindIII-spB(IgL)-s2/Hin3-CMV-IgHL-SVpA-a primer set using pmax+spacerB+IgL as a template. The amplification product was cloned into a pCR-Bluntll-TOPO vector, followed by cleaving with a restriction enzyme Hind III to obtain DNA sequence 13 (light chain cassette).

HindIII-spB(IgL)-s2 primer: 5'-ccttAAGCTTctattcctttgccctcggac-3' (SEQ ID No. 59)
Hin3-CMV-IgHL-SVpA-a primer: 5'-cttAAGCTTAACGTCTCGCCCTTTGGTCTC-3' (SEQ ID No. 60)

A basic vector+neo R plasmid was cleaved with a restriction enzyme Hind III and was inserted into DNA sequence 13 (light chain cassette) to obtain a basic vector+neoR+IgL plasmid.

### Acquisition of DNA fragment homologous to hprt gene of CHO cells

Genome DNA of CHO-K1 cells was provided as a template, and DNA fragments (CHA1 and CHA2) homologous to an hprt gene as a target were cloned by a PCR reaction (KOD-Plus-Ver2, TOYOBO). As shown in Fig. 3 which will be described later, CHA1 and CHA2 were set to sequences homologous to a region including exon 3 in the hprt gene. The following primer sequences were used in the PCR reaction.

SseNru-CHA1-1k-s primer: 5'-ttCCTGCAGGTCGCGAaggagtttattagaggaaatat-3' (SEQ ID No. 61)
MluI-CHA1-r primer: 5'-ttACGCGTtgataaaatctacagtcatggg-3' (SEQ ID No. 62)
Bam-CHA2-s primer: 5'-ttGGATCCgactgaagagctactgtgta-3' (SEQ ID No. 63)
PciNru-CHA2-1k-r primer: 5'- ttACATGTTCGCGAatcagatccctgggactgga-3' (SEQ ID No. 64)

### Acquisition of CHO 1kx2 antibody vector

A CHA2 sequence amplified with a Bam-CHA2-s/PciNru-CHA2-1k-r primer set was cleaved with restriction enzymes Bam HI and Pci I, followed by replacement with the HA2 sequence in the basic vector+neoR+IgL plasmid. Further, a CHA1 sequence amplified with an SseNru-CHA1-1k-s/Mlul-CHA1-r primer set was cleaved with restriction enzymes Sse8387I and Mlu I, followed by replacement with the HA1 sequence. Next, DNA sequence 8 (heavy chain cassette) was inserted into the restriction enzyme Mlu I site to obtain a CHO 1 kx2 antibody vector shown in Fig. 2 (A).

### Acquisition of HT1080 homology arm vector

DNA sequence 8 (heavy chain cassette) was inserted into a restriction enzyme Mlu I site in the basic vector+neoR+IgL plasmid to obtain an HT1080 homology arm vector shown in Fig. 2 (B).

### Example 3: Introduction of vector into cells

### Linearization of vector

The CHO 1kx2 antibody vector and the HT1080 homology arm vector were purified with Endofree Plasmid Maxi kit (QIAGEN) and were cleaved with Nru I, followed by dissolution in sterilized water to a concentration of 2 g/L, and the solutions were used in the following transfection experiment.

### Transfection and screening

CHO-K1 cells were adjusted with a nucleofection T solution (amaxa) to 1 × 10⁶ cells and were mixed with 2 µg of a linearized plasmid vector. Next, the mixed solution thus obtained and Nuceofector II (amaxa) were used to apply voltage with program U-023. The transfection was performed three times for each vector. The transfected cells were seeded in a 96 well plate at 350 cells/well and cultured in an incubator under conditions of 37°C and 5% CO₂ (medium: Advanced MEM (GIBCO) supplemented with 5%FBS, 1x Glutamax (GIBCO)), and, 2 days after the transfection, G418 (Invitrogen) was added (final concentration; 500 µg/mL).

After 4 days from the transfection, G418 (final concentration; 500µg/mL) and 6-TG (final concentration; 50µM) (Wako Pure Chemical Industries, Ltd.) were added, followed by culture for 6 days. After the culture, all the wells were confirmed, and G418/6TG-resistant colonies were isolated. The results were as shown in Table 1.

The results were as shown in Table 1. The number of G418/6TG-resistant clones obtained by the CHO 1kx2 antibody vector was 6 clones per 3 × 10⁶ cells. On the other hand, in the HT1080 homology arm vector, G418/6TG-resistant clones were not obtained.

**[Table 1]**

| Introduced vector | Number of G418/6TG-resistant clones acquired (per 3 × 10⁶ cells) | Number of integrated clones/number of clones as determined by genome PCR analysis | Number of integrated clones/number of clones as determined by Southern hybridization analysis |
|---|---|---|---|
| CHO 1 kb × 2 antibody vector | 6 | 5/6 | 5/5 |
| HT1080 homology arm vector | 0 | NOT TEST | NOT TEST |

### Example 4: Analysis by genome PCR

Genome DNA was extracted with DNA Isolation kit (Roche) from clones that were obtained by the transfection of the CHO 1 kx2 antibody vector and were resistant to G418 and 6TG. Recombination regiospecific to target hprt locus was confirmed by the following PCR reaction using the genome DNA as a template.

Fig. 3 (A) is a typical diagram for explaining details of an analysis of a homologous recombination reaction by genome PCR.
A target region (2) in homologous recombination of hprt gene (1) is set so as to contain exon 3 (3). CHA1 (4), CHA2 (5) and an antibody heavy chain sequence (7), a neomycin-resistant gene (8), and an antibody light chain sequence (9) held between CHA1 (4) and CHA2 (5) in the vector DNA (6) are integrated into this region by homologous recombination.

DNA (1688bp DNA) containing CHA1(4) and a part of the antibody heavy chain sequence (7) and DNA (1752bp DNA) containing CHA2 (5) and a part of the antibody light chain sequence (9) can be acquired from the genome only by homologous recombination and thus can be an index for homologous recombination.
Accordingly, a 1688bp DNA shown in Fig. 3 (A) was set as an index for homologous recombination between CHA1 and the target region, and the DNA was detected by a PCR reaction with primer CHPRTs/Notl-spacerA-s as shown below. On the other hand, 1752bp DNA shown in Fig. 3 (A) was set as an index for homologous recombination between CHA2 and the target hprt locus, and the DNA was detected by a PCR reaction with primer IGL-Cs/ CHA2-seq-a1 as shown below.

CHPRTs primer: 5'-TGTTCCTGTGCATACTAGGC-3' (SEQ ID No. 65)
CHA2-seq-a1 primer: 5'-CCAGAGAAATTATTTGCCACCAGC-3' (SEQ ID No. 66)

Next, the PCR amplification products thus obtained were analyzed by electrophoresis on 1.0% agarose gel. The results were as shown in Figs. 3 (B) and (C).
In Figs. 3 (B) and (C), numbers 1 to 6 represent clones obtained with the CHO 1kx2 antibody vector. Both the 1688bp and 1752bp PCR amplification products were confirmed in 5 clones among the acquired 6 clones, indicating that a homologous recombination reaction took place.

### Example 5: Analysis by Southern hybridization

The integration of the CHO 1kx2 antibody vector into the target hprt locus by regiospecific recombination in 5 clones acquired in Example 4 was confirmed by the following Southern hybridization.

Fig. 4 (A) is a typical diagram for explaining details of Southern hybridization in Example 5.
As shown in Fig. 4 (A), a target region (2) in homologous recombination of hprt gene (1) is set so as to contain exon 3 (3), and homology arm 1 (CHA1) (4) and homology arm 2 (CHA2) (5) are set so as to be homologous to two adjacent regions in the target region. Pci I restriction enzyme sites are located so as to hold the target region (2) therebetween and are absent in the target region (2). On the other side, the Pci I restriction enzyme site is absent in the CHO 1kx2 antibody vector. A neomycin-resistant gene sequence (6) in the CHO 1 kx2 antibody vector was designed so that an NR probe can be hybridized.

As shown in Fig. 4 (A), when 10488bp DNA has been detected, it is determined, from the position of the Pci I restriction enzyme sites and the chain length of the DNA sequence, that the CHO 1kx2 antibody vector has been integrated into the hprt locus of the clone.

### Manufacturing of NR probe

An NR probe having a sequence complementary to a neomycin-resistant gene in the CHO 1 kx2 antibody vector was synthesized according to the following procedure. At the outset, the entire length of a neomycin-resistant gene coding sequence in the CHO 1kx2 antibody vector was amplified by PCR, followed by TA cloning into a pGEM T plasmid vector (Promega). Next, a DIG (Digoxigein) labeled probe was prepared with a PCR DIG probe synthesis kit (Roche, primer: M13 Forward/Reverse primer).

### Preparaton of membrane

Genome DNA of each cell clone was extracted from the 6TG resistant colony and was cleaved with a Pci I restriction enzyme. The cleaved genome DNA (5 µg) was electrophoresed on 0.6% agarose gel and blotted onto a nylon membrane (Hybond N+ membrane, Amaersham Biosciences). The membrane thus obtained was incubated at 80°C for 2 hr to immobilize DNA on the membrane.

### Hybridization

The membrane was hybridized with the NR probe. In this case, prehybridization, hybridization and probe detection were carried out according to a DIG Application Manual (Roche).

As shown in Fig. 4 (B), in all of the analyzed 5 clones, a 10488bp DNA fragment was detected by the NR probe.

### Example 6: Production of protein by recombinant cells

### Culture of recombinant clone and sampling of medium

At the outset, the recombinant clone was seeded in an amount of 2 x 10⁵ cells per plate and was cultured in 10 mL of Advanced MEM (Invitrogen) containing G418 (final concentration; 500 µg/mL), 6-TG (final concentration; 50 µM), and FBS (Japan Bio Serum) (5%) in the presence of 5% CO₂ at 37°C. On the fifth day from the start of the culture, the medium was collected. The collected medium was used for the following ELISA analysis.

### Quantitative determination by ELISA

The amount of IgG in the collected medium was determined by measuring the absorbance at 450 nm with Human IgG EIA Kit (precoated) (Takara Shuzo Co., Ltd.).

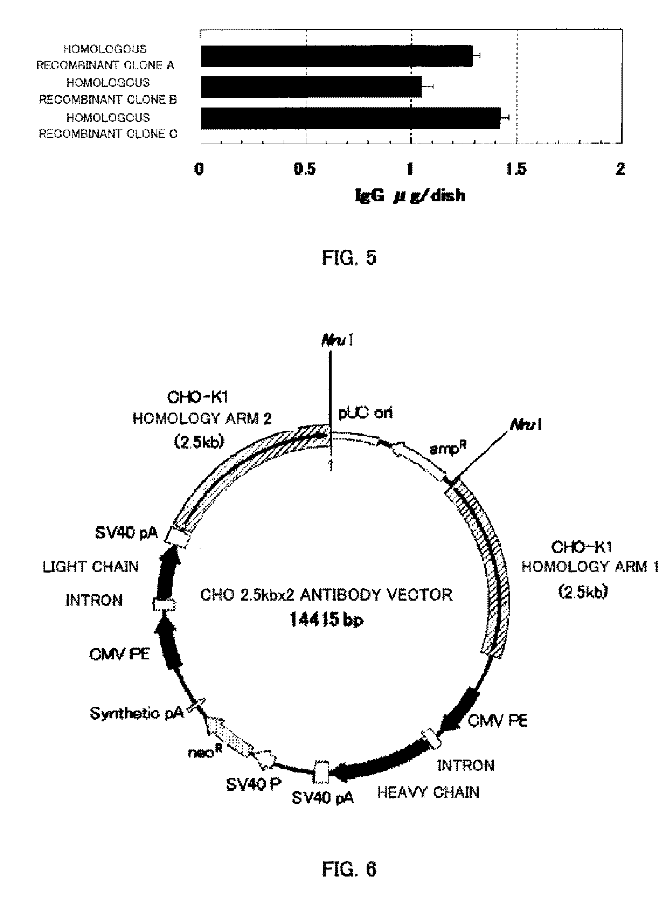

The results were as shown in Fig. 5. In the measured 3 clones, the amount of IgG per plate was 1.29±0.18µg/dish on average, and the CV value was 14.2%.

### Example 7: Homologous recombination with vector having homologous DNA fragment having chain length of 2.5 kbp

### Acquisition of 2.5-kbp DNA fragment homologous to hprt gene in CHO cells

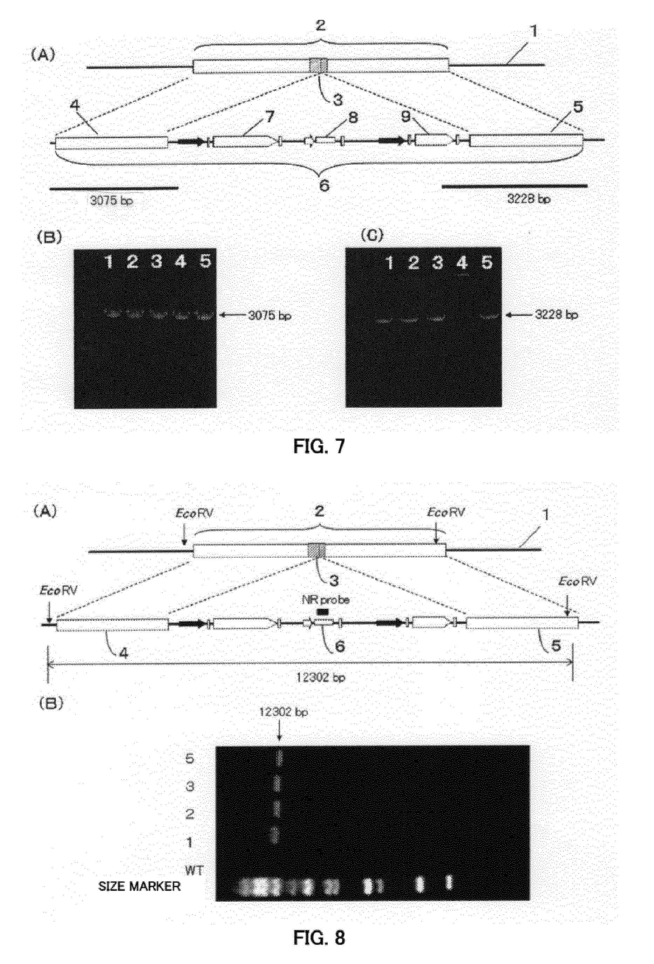

In order to manufacture a CHO 2.5kx2 antibody vector that has a homologous DNA fragment having a chain length of 2.5 kbp and is shown in Fig. 6, DNA fragments homologus to target hprt gene (CHA1-2.5 and CHA2-2.5) were cloned by a PCR reaction (KOD-Plus-Ver2, TOYOBO) using genome DNA of CHO-K1 cells as a template. As shown in Fig. 7 (A) which will be described later, CHA1-2.5 and CHA2-2.5 were set to a sequence homologous to a region containing exon 3 of the hprt gene. Mlul-CHA1-r primer and Bam-CHA2-s primer described in Example 2 and the following primers were used as primer sequences in the PCR reaction.

SseNru-CHA1-2.5k-s primer: 5'-ttCCTGCAGGTCGCGAgtctgtgtgtatgtttgtgataggc-3' (SEQ ID No. 67)
NcoNru-CHA2-2.5k-r primer: 5'-ttCCATGGTCGCGAtgaaggttatagagcataggggacc-3' (SEQ ID No. 68)

### Acquisition of CHO 2.5kx2 antibody vector

The CHA2-2.5 sequence amplified with the Bam-CHA2-s/NcoNru-CHA2-2.5k-r primer set was cleaved with restriction enzymes Bam HI and Nco I, followed by replacement with the HA2 sequence in the basic vector+neo R+IgL plasmid. Further, the CHA1-2.5 sequence amplified with the SseNru-CHA1-2.5k-s/MluI-CHA1-r primer set was cleaved with restriction enzymes Sse8387I and Mlu I, followed by replacement with the HA1 sequence. Next, DNA sequence 8 (heavy chain cassette) was inserted into the restriction enzyme Mlu I site to obtain a CHO 2.5kx2 antibody vector shown in Fig.6.

### Acquisition of CHO 2.5kx2 antibody vector integrated clone

The CHO 2.5kx2 antibody vector was purified and linearized in the same manner as in Example 3. The linearized vector was dissolved in sterilized water to a concentration of 2 g/L, followed by introduction into 1 × 10⁶ cells.

Next, screening with a selective drug was carried out in the same manner as in Example 3. As a result, G418/6TG-resistant colonies of 5 clones were isolated.

### Analysis by genome PCR

Genome DNA was extracted from clones resistant to G418 and 6TG obtained by the transfection of the CHO 2.5kx2 antibody vector, and recombination regiospecific to the target hprt locus was confirmed by the following PCR reaction using the genome DNA as a template.

Fig. 7 (A) is a typical diagram for explaining details of an analysis of a homologous recombination reaction by genome PCR.
A target region (2) of homologous recombination in an hprt gene (1) is set so as to contain exon 3 (3). CHA1-2.5 (4), CHA2-2.5 (5), an antibody heavy chain sequence (7), a neomycin-resistant gene (8), and an antibody light chain sequence (9) held between CHA1-2.5 (4) and CHA2-2.5 (5) in a vector DNA (6) are integrated into this region by homologous recombination.

DNA (3075-bp DNA) containing CHA1-2.5 (4) and a part of the antibody heavy chain sequence (7) and DNA (3228-bp DNA) containing CHA2-2.5 (5) and a part of the antibody light chain sequence (9) can be acquired from the genome only by homologous recombination and thus can be an index for homologous recombination.
Accordingly, a 3075-bp DNA shown in Fig. 7 (A) was set as an index for homologous recombination between CHA1-2.5 and the target region, and the DNA was detected by a PCR reaction with primer CHA1-seq-s11/Notl-spacerA-s. On the other hand, 3228-bp DNA shown in Fig. 7 (A) was set as an index for homologous recombination between CHA2-2.5 and the target hprt locus, and the DNA was detected by a PCR reaction with primer IGL-Cs/ CHA2-seq-a4.

CHA1-seq-s11 primer: 5'-GACACATGCAGACAGAACAG-3' (SEQ ID No. 69)
CHA2-seq-a4 primer: 5'-GTTTGCTAACACCCCTTCTC-3' (SEQ ID No. 70)

Next, the PCR amplification products thus obtained were analyzed by electrophoresis on 1.0% agarose gel. The results were as shown in Figs. 7 (B) and (C).
In Figs. 7 (B) and (C), numbers 1 to 5 represent clones obtained with the CHO 2.5kx2 antibody vector. Both the 3075-bp and 3228-bp PCR amplification products were confirmed in 4 clones among the acquired 5 clones, indicating that a homologous recombination reaction took place.

### Analysis by Southern hybridization

The regiospecific integration of the CHO 2.5kx2 antibody vector into the target hprt locus in 4 clones acquired in Example 7 was confirmed by the following Southern hybridization.

Fig. 8 (A) is a typical diagram for explaining details of Southern hybridization in Example 7
As shown in Fig. 8 (A), a target region (2) in homologous recombination of hprt gene (1) is set so as to contain exon 3 (3), and homology arm 1 (CHA1-2.5) (4) and homology arm 2 (CHA2-2.5) (5) are set so as to be homologous to two adjacent regions in the target region. Only one Eco RV restriction enzyme site is present around the target region (2), and only one Eco RV restriction enzyme site is present within the target region. On the other hand, in the CHO 2.5kx2 antibody vector, only one Eco RV restriction enzyme site is present in CHA2-2.5.. A neomycin-resistant gene sequence (6) in the CHO 2.5kx2 antibody vector was designed so that an NR probe can be hybridized.

As shown in Fig. 8 (A), when 12302-bp DNA has been detected, it is determined, from the position of the Eco RV restriction enzyme sites and the chain length of the DNA sequence, that the CHO 2.5kx2 antibody vector has been integrated into the hprt locus of the clone.

### Manufacturing of probe

An NR probe having a sequence complementary to a neomycin-resistant gene in the CHO 2.5kx2 antibody vector was provided in the same manner as in Example 5.

### Preparation of membrane

Genome DNA of each cell clone was extracted from the 6TG resistant colony and was cleaved with an Eco RV restriction enzyme. The cleaved genome DNA was subjected to a Southern hybridization analysis in the same manner as in Example 5.

As shown in Fig. 8 (B), in all of the analyzed 4 clones, a 12302-bp DNA fragment was detected by the NR probe.

### Example 8: Confirmation of homologous recombination in two chromosomes of CHO cells

### Confirmation of remained wild-type hprt gene by genome PCR

Whether or not a wild-type hprt gene remains was confirmed by the following PCR reaction using genome DNA extracted from homologous recombination clones of the CHO 1kx2 antibody vector and the CHO 2.5kx2 antibody vector as a template.

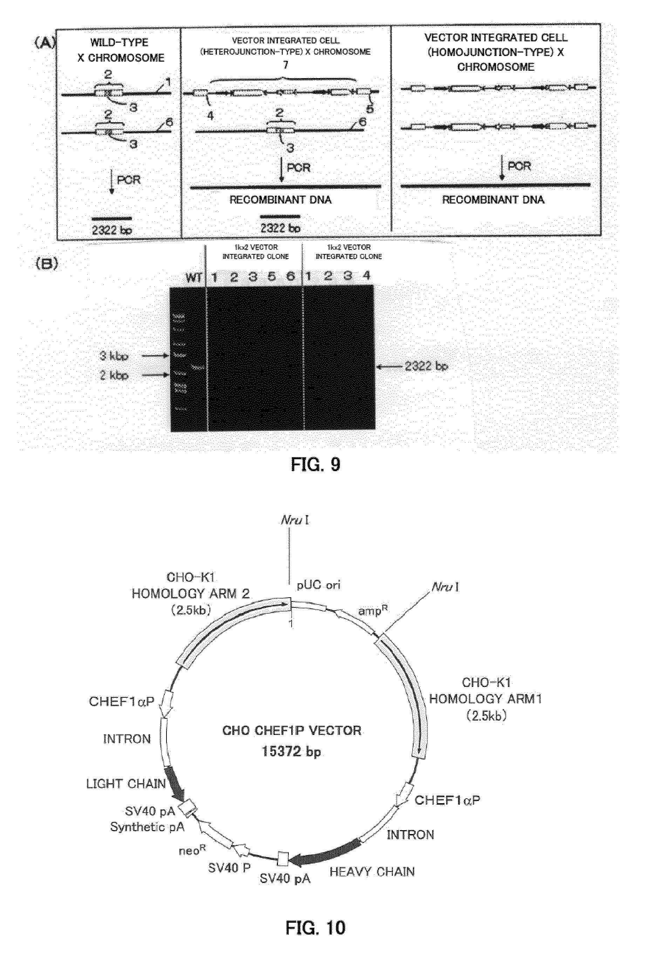

Fig. 9 (A) is a typical diagram for explaining details of genome PCR for the confirmation of remained wild-type hprt gene.
At the outset, the left drawing in Fig. 9 (A) shows PCR in wild-type CHO cells having two X chromosomes. A target region (2) in homologous recombination of an hprt gene (1) is set so as to contain exon 3 (3). In vector non-introduced wild-type cells, DNA (2322 bp) of the target region can be amplified by a PCR reaction, and, thus, the remaining of the wild-type hprt gene can be confirmed using this as an index.

The drawing located at the center of Fig. 9 (A) shows PCR in heterojunction-type homologous recombinant cells comprising a vector integrated into an hprt locus of one X chromosome. In one chromosome, vector DNA (7) (containing CHA1(4), CHA2(5), and an antibody heavy chain, a neomycin-resistant gene, and an antibody light chain sequence held between CHA1(4) and CHA2(5)) is integrated into the target region (2) by homologous recombination. On the other hand, another chromosome holds a target region, and DNA (2322 bp) in the target region can be amplified by a PCR reaction, making it possible to confirm the remaining of the wild-type hprt gene.

On the other hand, as shown in the drawing located right in Fig. 9 (A), in homojunction-type homologous recombinant cells comprising a vector integrated into both X chromosomes, only recombinant DNA is expressed, and DNA (2322 bp) in the target region is not amplified by the PCR reaction.

Based on the foregoinig understanding, the 2322-bp DNA shown in Fig. 9 (A) was set as the index, and, for the DNA detection, a PCR reaction was carried out with primer CHPRTs/CHA2-seq-a1.
Next, the PCR amplification product thus obtained was analyzed by electrophoresis on 1.0% agarose gel. The results were as shown in Fig. 9 (B).

In Fig. 9 (B), WT represents wild-type cells, 1kx2 vector integrated clones (1, 2, 3, 5, and 6) represent CHO 1kx2 antibody vector integrated clones acquired in Example 4, and 2.5kx2 vector integrated clones (1 to 4) represent CHO 2.5kx2 antibody vector integrated clones acquired in Example 7. 2322-bp PCR amplification products were confirmed from the wild-type cells, and, thus, the presence of the wild-type hprt gene was confirmed. On the other hand, in all the analyzed recombinant clones, any 2322-bp PCR amplification product was not confirmed, indicating that the recombinant cells were of a homojunction type in which a vector was integrated into both X chromosomes.

From the results, it was confirmed that the use of the vector according to the present invention allowed recombinant clones comprising a target protein gene integrated into both hprt locuses of two X chromosomes to be acquired with high frequency.

### Example 9: Long-term stable expression of antibody by recombinant vector clones using CHO endogenous promoter

### Manufacturing of CHO CHEF1P vector

A CHEF1α promoter was amplified with an EF1 aP-Mlul-F/EF1 aP-Xbal-R primer set using a CHO-K1 genome DNA as a template.

EF1aP-Mlul-F primer: 5'-AGAACGCGTCCACACAATCAGAACCACA-3' (SEQ ID No. 71)
EF1aP-Xbal-R primer: 5'-GACGATCTAGAGGTGGTTTTCACAACA-3' (SEQ ID No. 72)

Further, an IgH gene was amplified with a CMV-seq-s/MluI-CMV-IgHL-SVpA-a primer set using a CHO 2.5k×2_H-neo-L plasmid as a template.

CMV-seq-s primer: 5'-AGGGACTTTCCATTGACGTC-3' (SEQ ID No. 73)
MluI-CMV-IgHL-SVpA-a primer: 5'-cttACGCGTAACGTCTCGCCCTTTGGTCTC-3' (SEQ ID No. 74)

Next, the CHEF1αP and IgH gene thus obtained were cleaved with Xbal and Nhel, respectively, followed by ligation. Thereafter, a band of the CHEF1αP+IgH gene was subjected to gel extraction, and the CHEF1αP+IgH gene was cloned into a TOPO vector (Invitrogen), followed by cleaving with MluI to obtain a CHEF1αP+IgH gene cassette.

On the other hand, a CHEF1α promoter was amplified with an EF1aP-HindIII-F/EF1aP-HindIII-R primer set using CHO-K1 genome DNA as a template.

EF1aP-HindIII-F primer: 5'-AGAAAGCTTCCACACAATCAGAACCACA-3' (SEQ ID No. 75)
EF1aP-HindIII-R primer: 5'-GACGAATTCGCGGTGGTTTTCACAACA-3' (SEQ ID No. 76)

An IgL gene was amplified with an IgLser-EcoRI-F/IgLser-HindIII-R primer set using a CHO 2.5k×2_H-neo-L plasmid as a template.

IgLser-EcoRI-F primer: 5'-TATGAATTCGTCGCCACCATGGACAT-3' (SEQ ID No. 77)
IgLser-HindIII-R primer: 5'-TGTAAGCTTTACCACATTTGTAGAGGTTTT-3' (SEQ ID No. 78)

The CHEF1αP and IgL gene thus obtained were cleaved with EcoRI, followed by ligation. Thereafter, a band of the CHEF1αP+IgL gene was subjected to gel extraction, and the CHEF1αP+IgL gene was cloned into a TOPO vector, followed by cleaving with HindIII to obtain a CHEF1αP+IgL gene cassette.

The CHO 2.5k×2_H-neo vector was cleaved with HindIII and was dephosphorylated, and the CHEF1αP+IgL gene cassette was inserted thereinto to obtain an H-neo-EF1L vector. Next, after MluI cleaving and dephosphorylation, the CHEF1αP+IgH gene cassette was inserted thereinto to obtain a CHO CHEF1P vector shown in Fig. 10.

### Acquisition of CHO CHEF1P vector recombinant CHO cells

Linearization, introduction into CHO cells, and screening of the constructed CHO CHEF1P vector were carried out in the same manner as in Example 3 to acquire CHO CHEF1 P vector recombinant CHO cells.

### Confirmation of long-term stability in antibody production of CHO CHEF1P vector clone

The acquired CHO CHEF1P vector recombinant CHO cells were subcultured (selective drug-free medium: Advanced MEM (GIBCO) supplemented with 5% FBS, 1xGlutamax (GIBCO)) under conditions of 7°C and 5% CO₂ and were used in a timely manner for the measurement of the amount of antibody production.

Cells during subculture were peeled off, were seeded at 2×10⁶ cells in a 100-mm dish, and were cultured under conditions of 37°C and 5% CO₂ overnight. The cultured cells were peeled off and adjusted to 3×10⁵ cells/ml. The cells were seeded in a 12-well plate in an amount of 1.5 ml per well and were cultured. After the elapse of 24 hr from the start of the culture, the medium was entirely removed. A fresh medium (1.5 ml) was dispensed, followed by culture for 24 hr. The whole quantity of the medium was collected, and the amount of IgG was measured by ELISA assy. Cells adhered on the bottom of the wells were peeled off, and the number of cells was counted.

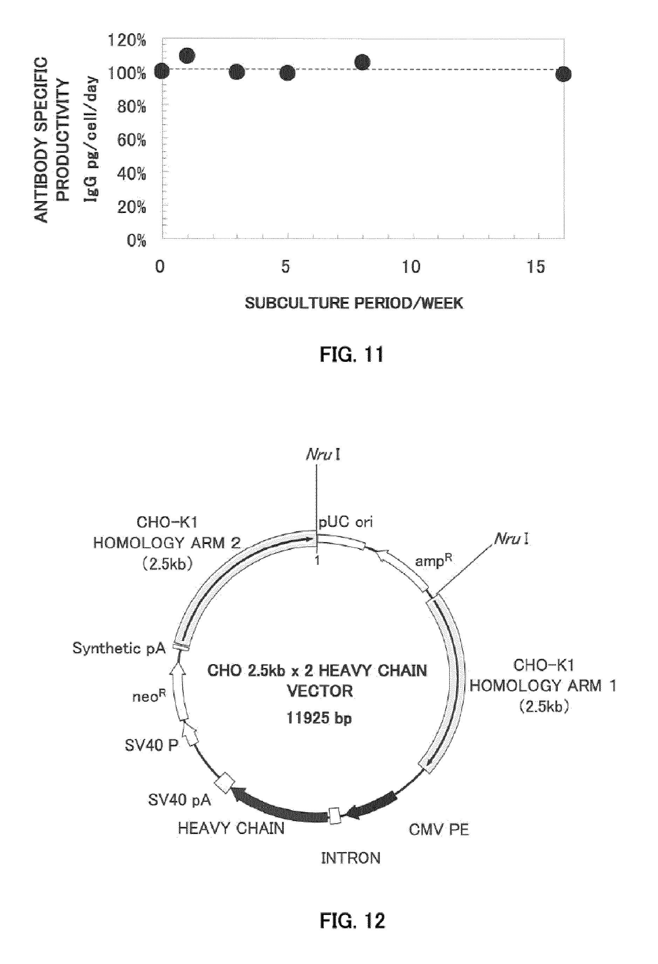

The results were as shown in Fig. 11. The antibody productivity of CHO cells comprising a CHO CHEF1 P vector integrated into an hprt locus was kept constant even after subculture for 16 weeks under selective drug-free conditions, and the productivity was not lowered.

### Example 10: Acquisition of homologous recombinant by vector that has homologous DNA fragment having chain length of 2.5 kbp and expresses antibody heavy chain

### Manufacturing of recombinant vector that expresses antibody heavy chain

The CHO 2.5kx2 antibody vector prepared in Example 7 was cleaved with a restriction enzyme Hind III and was subjected to slef-ring closing to prepare a CHO 2.5kbx2 heavy chain vector shown in Fig. 13.

### Acquisition of CHO 2.5kbx2 heavy chain vector recombinant CHO cells

Linearlization, introduction into CHO cells, and screening of the constructed CHO 2.5kbx2 heavy chain vector were carried out in the same manner as in Example 3 to acquire CHO 2.5kbx2 heavy chain vector recombinant CHO cells. As a result, 130 recombinant CHO cells per 10⁷ cells were acquired.

### Example 11: Acquisition of stable expression CHO cells by vector that has MAR derived from human hprt locus intron and expresses antibody heavy chain

### Manufacturing of MAR non-transcriptional vector

MAR (hprtMAR1) of a human hprt gene intron was amplified with a Xbal-hprtMAR-s/Xbal-hprtMAR-a primer set using the genome DNA of an HT1080 cell line, a human-derived cell line, provided in Example 2 as a template.

Xbal-hprtMAR-s primer: 5'-ttTCTAGAtagttatgagcccatgtccc-3' (SEQ ID No. 79)
Xbal-hprtMAR-a primer: 5'-ttTCTAGAcggtgaaatcctgtctctac-3' (SEQ ID No. 80)

The CHO 2.5kx2 antibody vector prepared in Example 7 was cleaved with an restriction enzyme Xbal, and hprtMAR1 was inserted thereinto to obtain an MAR non-transcriptional vector shown in Fig. 13 (A).

### Manufacturing of MAR transcriptional vector

MAR (hprtMAR2) of a human hprt gene intron was amplified with an Ascl-hprtMAR-s/MluI-hprtMAR-a primer set using genome DNA of an HT1080, a human-derived cell line, as a template.

Ascl-hprtMAR-s primer: 5'-ttGGCGCGCCtagttatgagcccatgtccc-3' (SEQ ID No. 81)
MluI-hprtMAR-a primer: 5'-ttACGCGTcggtgaaatcctgtctctac-3' (SEQ ID No. 82)

The CHO 2.5kx2 antibody vector prepared in Example 7 was cleaved with a restriction enzyme Mlul, and hprtMAR2 was inserted thereinto. Next, the product was cleaved with a restriction enzyme Mlul, and the antibody heavy chain expression cassette of Example 2 was inserted thereinto to obtain an MAR transcriptional vector shown in Fig. 13 (B).

### Manufacturing of MAR CMV transcriptional vector

MAR (hprtMAR3) of a human hprt gene intron was amplified with a BgIII-hprtMAR-s/BgIII-hprtMAR-a primer set using genome DNA of an HT1080, a human-derived cell line, as a template.

BgIII-hprtMAR-s primer: 5'-ttAGATCTtagttatgagcccatgtccc-3' (SEQ ID No. 83)
BgIII-hprtMAR-a primer: 5'-ttAGATCTcggtgaaatcctgtctctac-3' (SEQ ID No. 84)

The pCR-BluntII-TOPO+antibody heavy chain expression cassette prepared in Example 2 was cleaved with a restriction enzyme BgIII, and hprtMAR3 was inserted thereinto. The product was taken off with a restriction enzyme Mlul to obtain an antibody heavy chain +MAR expression cassette.

The pCR-BluntII-TOPO+antibody light chain expression cassette prepared in Example 2 was cleaved with a restriction enzyme BgIII, and hprtMAR3 was inserted thereinto. The product was taken off with a restriction enzyme HindIII to obtain an antibody light chain +MAR expression cassette.

The CHO 2.5kx2 antibody vector prepared in Example 7 was cleaved with a restriction enzyme Mlul, and an antibody heavy chain +MAR expression cassette was inserted thereinto. Next, the product was cleaved with a restriction enzyme Hindlll, and an antibody light chain +MAR expression cassette was inserted thereinto to obtain an MAR CMV transcriptional vector shown in Fig. 13 (C).

### Acquisition of MAR vector recombinant CHO cells

Linearization, introduction into CHO cells, and screening of the constructed MAR non-transcriptional vector, MAR transcriptional vector and MAR CMV transcriptional vector were carried out in the same manner as in Example 3 to obtain recombinant CHO cells.

### Confirmation of stability in antibody production of vector clones that have MAR derived from human hprt locus intron and expresses antibody heavy chain

The analysis of antibody productivity of recombinant CHO clones acquired by MAR vector shown in Figs. 13 (A), (B), and (C) was carried out in the same manner as in Example 9.

The results were as shown in Fig. 14. As is apparent from the results, clones that could maintain antibody productivity were only clones with a vector into which MAR of human hprt is transcribed only by transcription of the hprt gene at the vector integration site being integrated thereinto. For clones acquired by a vector having a human hprtMAR non-transcriptional structure and a vector having a structure that the target protein gene and MAR are simultaneously transcribed by a CMV promoter, the antibody productivity was lowered during culture for 7 days.

### Example 12: Acquisition of homologous recombinant by vectors that have homologous DNA fragments having chain lengths of 200b and 500b and express antibody heavy chain

### Manufacturing of CHO 200b×2 vector and CHO 500b×2 vector

Homologous DNA fragments (CHA1-200 and CHA2-200) of target hprt gene were cloned by a PCR reaction respectively with an SseNru-CHA1-200-s/Mlul-CHA1-r primer set and a Bam-CHA2-s/PciNru-CHA2-200-r primer set using genome DNA of CHO-K1 cells as a template. CHA1-200 and CHA2-200 were set to a sequence homologous to a region containing exon 3 of an hprt gene. The following primer sequences were used in the PCR reaction.

SseNru-CHA1-200-s primer: 5'-ttCCTGCAGGTCGCGAtggaatcttctattcctgattt-3' (SEQ ID No. 85)
PciNru-CHA2-200-r primer: 5'-ttACATGTTCGCGAtcagcactcaggagtcagag-3' (SEQ ID No. 86)

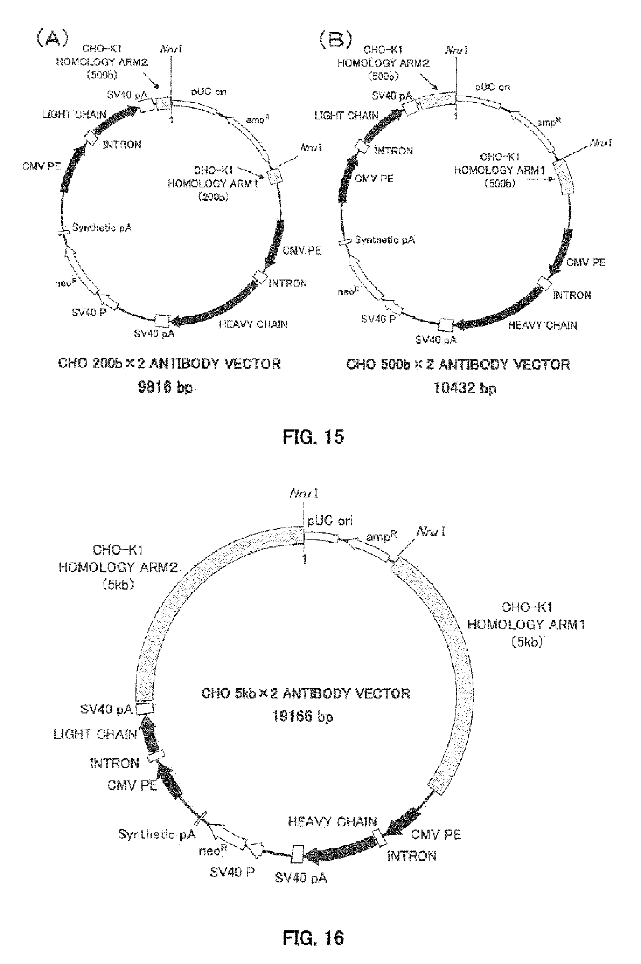

The basic vector+neoR+IgL plasmid of Example 2 was cleaved with restriction enzymes Bam HI and Pci I, followed by replacement with the CHA2-200 fragment. The product was then cleaved with restriction enzymes Sse8387I and Mlu I, followed by replacement with the CHA1-200 fragment. Finally, DNA sequence 8 (heavy chain cassette) was inserted into the restriction enzyme Mlu I site to obtain a CHO 200b×2 vector having a homology arm length of 200b shown in Fig. 15 (A).

On the other hand, homologous DNA fragments of a target hprt gene (CHA1-500 and CHA2-500) were cloned by a PCR reaction respectively with an SseNru-CHA1-500-s/MluI-CHA1-r primer set and a Bam-CHA2-s/PciNru-CHA2-500-r primer set.

SseNru-CHA1-500-s primer: 5'-ttCCTGCAGGTCGCGAgatgcctagcatgtacctgg-3' (SEQ ID No. 87)
PciNru-CHA2-500-r primer: 5'-ttACATGTTCGCGAtaagtacaaatccatcttgggtgac-3' (SEQ ID No. 88)

Next, the basic vector+neo R+IgL plasmid of Example 2 was cleaved with restriction enzymes Bam HI and Pci I, followed by replacement with the CHA2-500 fragment. Next, the product was cleaved with restriction enzymes Sse8387I and Mlu I, followed by replacement with the CHA1-500 fragment. Finally, DNA sequence 8 (heavy chain cassette) was inserted into the restriction enzyme Mlu I site to obtain a CHO 500b×2 vector having a homology arm length of 500b shown in Fig. 15 (B).

### Acquisition of CHO 200b×2 vector and CHO 500b×2 vector recombinant CHO cells

Linearization, introduction into CHO cells, and screening of the constructed CHO 200b×2 vector and CHO 500b×2 vector were carried out in the same manner as in Example 3.

Despite the fact that the introduction operation was carried out twice, recombinant CHO cells were not acquired at all in any of the CHO 200b×2 vector and the CHO 500b×2 vector.

### Example 13: Acquisition of homologous recombinant by vector that has homologous DNA fragment having chain length of 5 kb and expresses antibody heavy chain

### Manufacturing of CHO 5kb×2 vector

Homologous DNA fragments of a target hprt gene (CHA1-5k and CHA2-5k) were cloned by a PCR reaction respectively with an NsiNru-CHA1-5k-s/MluI-CHA1-r primer set and a BcII-CHA2-s/BspNru-CHA2-5k-r primer set using genome DNA of CHO-K1 cells as a template. CHA1-5k and CHA2-5k were set to a sequence homologous to a region containing exon 3 of the hprt gene. The following primer sequences were used in the PCR reaction.

NsiNru-CHA1-5k-s primer: 5'-ttATGCATTCGCGAAtctcaggtgataggagacataagac-3' (SEQ ID No. 89)
Bcll-CHA2-s primer: 5'-ttTGATCAgactgaagagctactgtgta-3' (SEQ ID No. 90)
BspNru-CHA2-5k-r primer: 5'-ttTCATGAaTCGCGAAtcagcactcaggagtcagag-3' (SEQ ID No. 91)

Next, the basic vector+neo R+IgL plasmid of Example 2 was cleaved with restriction enzymes Bam HI and Pci I, followed by replacement with the CHA2-5k fragment. Next, the product was cleaved with restriction enzymes Sse8387I and Mlu I, followed by replacement with the CHA1-5k fragment. Finally, DNA sequence 8 (heavy chain cassette) was inserted into the restriction enzyme Mlu I site to obtain a CHO 5kb×2 vector having a homology arm length of 5 kb shown in Fig. 16.

### Acquisition of CHO 5kb×2 vector recombinant CHO cells

Linearization, introduction into CHO cells, and screening of the constructed CHO 5kb×2 vector were carried out in the same manner as in Example 3.
As a result, recombinant CHO cells of 7 clones per 1×10⁶ cells were acquired. The frequency of acquisition with the vector having an homology arm length of 2.5 kb of Example 7 was 4 clones per 1 ×10⁶ cells, indicating that the homomogy arm length of 5 kb provided better results.

## Claims

1. A DNA construct comprising, from a 5' end toward a 3' end, a first homologous DNA fragment, a target protein gene, and a second homologous DNA fragment,
wherein the first and second homologous DNA fragments have homology allowing for homologous recombination with a part of a hypoxanthine-phosphoribosyltransferase enzyme (hprt) locus in a CHO cell genome and having a chain length of not less than 1 kbp.

2. The DNA construct according to claim 1, wherein the first and second homologous DNA fragments have a chain length of 1 kbp to 7.5 kbp.

3. The DNA construct according to claim 1, wherein the first and second homologous DNA fragments have a chain length of 1 kbp to 5 kbp.

4. The DNA construct according to claim 1, wherein the part of the hprt locus is a region which includes at least a part of introns of the hprt gene.

5. The DNA construct according to claim 1, wherein the first or second homologous DNA fragment includes a base sequence described in any one of SEQ ID Nos 15 to 22 or a partial sequence thereof.

6. The DNA construct according to claim 1, wherein the target protein is an antibody, an enzyme, a cytokine, a hormone, a coagulation factor, a regulatory protein, or a receptor.

7. The DNA construct according to claim 1, which further comprises a positive selective marker gene.

8. A vector comprising a DNA construct according to claim 1.

9. The vector according to claim 8, wherein a nuclear/matrix adhesion region (MAR) derived from a first intron of a human hprt gene is maintained such a structure that, after the integration of the vector into a chromosome, the MAR per se is transcribed by transcription at the integration site.

10. The vector according to claim 8 or 9, which comprises a CHEF-1α gene promoter operatively linked to the target protein gene.

11. The vector according to any one of claims 8 to 10, wherein the vector is a plasmid vector, a cosmid vector, a phage vector, or an artificial chromosome vector.

12. A process for generating a recombinant CHO cell, the process comprising introducing the vector according to any one of claims 8 to 10 into CHO cells.

13. A recombinant CHO cell comprising an exogenous target protein gene integrated into an hprt locus.

14. The recombinant CHO cell according to claim 13, wherein the function of the hprt gene has been inactivated.

15. A recombinant CHO cells generated by a process according to claim 12.

16. A process for producing a target protein, the process comprising:
providing the recombinant CHO cell according to claim 13 or 15; and culturing the cell to produce a target protein.
